(19) Europäisches Patentamt
European Patent Office
Office européen des brevets

(11) EP 4 549 540 A1

(12) EUROPEAN PATENT APPLICATION

(43) Date of publication:
**07.05.2025 Bulletin 2025/19**

(21) Application number: **23207441.9**

(22) Date of filing: **02.11.2023**

(51) International Patent Classification (IPC):
***C11D 3/00*** *(2006.01)* ***C11D 3/37*** *(2006.01)*
***C11D 3/22*** *(2006.01)* ***C11D 3/48*** *(2006.01)*

(52) Cooperative Patent Classification (CPC):
**C11D 3/0036; C11D 3/0068; C11D 3/227; C11D 3/3715; C11D 3/48;** C11D 2111/12

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **The Procter & Gamble Company**
**Cincinnati, OH 45202 (US)**

(72) Inventors:
- **LIEDEL, Clemens**
  **65926 Frankfurt am Main (DE)**
- **MCDONNELL, Michael**
  **Newcastle upon Tyne, NE12 9BZ (GB)**
- **RIDLEY, Lauren**
  **Newcastle upon Tyne, NE12 9BZ (GB)**
- **SCHAEFER, Carsten**
  **65926 Frankfurt am Main (DE)**
- **SI, Gang**
  **Newcastle upon Tyne, NE12 9BZ (GB)**
- **SIVIK, Mark**
  **Cincinnati, 45202 (US)**
- **YAMADA, Hiroe**
  **65926 Frankfurt am Main (DE)**

(74) Representative: **P&G Patent Belgium UK**
**N.V. Procter & Gamble Services Company S.A.**
**Temselaan 100**
**1853 Strombeek-Bever (BE)**

# (54) FABRIC AND HOME CARE COMPOSITION

(57) The present invention relates to a fabric and home care composition containing a fabric and home care ingredient, an anionic polyester soil release polymer and a polysaccharide-based cationic polymer.

EP 4 549 540 A1

## Description

### FIELD OF THE INVENTION

The present invention relates to a fabric and home care composition comprising an anionic soil release polymer and a polysaccharide-based cationic polymer.

### BACKGROUND OF THE INVENTION

Soil release polymers are known and used in fabric and home care formulations. In the washing process, a soil release polymer can deposit on fibers, which change the surface properties of fabric and deliver various benefits, such as reduced soil deposition onto fabric during wash and wear; reduced adhesion of microorganism and allergens onto fabric; easier soil removal from fabrics which treated with soil release polymer in previous wash; reduced malodor; improved wicking properties.

Cationic polymers are typically incorporated into fabric and home care formulations to provide care benefits.

Anionic soil release polymers typically have compatibility issues with cationic polymers when they are formulated together. The present invention addresses the problem by providing a fabric and home composition comprising cationic polymer and a specific anionic soil release polymer. The compositions of the present invention provide both good cleaning performance as well as good care performance.

In particular, the composition of the present invention can reduce the adhesion of soil to a fabric surface, reduce the adhesion of biological stains or microorganisms to textiles, control malodor, and deposit one or more depositable conditioning active onto a surface.

### SUMMARY OF THE INVENTION

The present invention provides a fabric and home care composition comprising:

(a) a fabric and home care ingredient;
(b) an anionic polyester soil release polymer; and
(c) a polysaccharide-based cationic polymer,

wherein the anionic polyester soil release polymer comprises:

(A) one or more structural units of the formula (I)

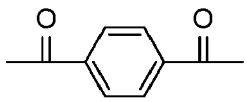

(I)

and
(B) one or more structural units of the formula (II)

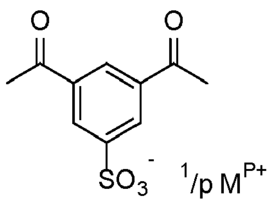

(II)

wherein
$^{1}/_{p}\, M^{p+}$ is a cation, preferably selected from the group consisting of monovalent cations $M^+$ (p = 1), divalent

cations ½ $M^{2+}$ (p = 2) and trivalent cations $^{1}/_{3}$ $M^{3+}$ (p = 3) and more preferably selected from the group consisting of $H^+$, $Li^+$, $Na^+$, $K^+$, ½ $Mg^{2+}$, ½ $Ca^{2+}$, $^{1}/_{3}\ Al^{3+}$, $NH_4^+$ and $R^aR^bR^cR^dN^+$, wherein $R^a$, $R^b$, $R^c$ and $R^d$, independently of one another, are H, linear or branched, preferably linear, ($C_1$-$C_{22}$)-alkyl groups or linear or branched, preferably linear, ($C_2$-$C_{10}$)-hydroxyalkyl groups, and wherein in the cations $R^aR^bR^cR^dN^+$ at least one of $R^a$, $R^b$, $R^c$ and $R^d$ is not H, and
(C) one or more structural units of the formula (III)

$$-O-R^1-O- \qquad (III)$$

wherein

$R^1$ is a linear or branched alkylene group represented by the formula ($C_mH_{2m}$) wherein m is an integer from 2 to 12, preferably from 2 to 6, more preferably from 2 to 4 and even more preferably from 2 to 3, most preferably 3,

and
(D) one or more terminal groups of the formula (IV)

$$-O-[C_nH_{2n}-O]_x-R^2 \qquad (IV)$$

wherein

$R^2$ is a linear or branched $C_1$-$C_{30}$ alkyl group, a cycloalkyl group with 5 to 9 carbon atoms or a $C_6$-$C_{30}$ arylalkyl group, preferably a linear or branched $C_1$-$C_{30}$ alkyl group, more preferably a linear $C_1$-$C_6$ alkyl group and even more preferably $CH_3$,

n is 2 or an integer > 2, preferably is an integer from 2 to 12, more preferably is an integer from 2 to 6 and even more preferably is an integer from 2 to 4, whereby the definition of n may vary within a single terminal group of the formula (IV), and

x is, based on molar average, a number of at least 30, preferably from 30 to 200, more preferably from 40 to 180, even more preferably from 50 to 150, particularly preferably from 60 to 120 and extraordinarily preferably from 65 to 115.

## DETAILED DESCRIPTION OF THE INVENTION

The fabric and home care composition comprises an anionic polyester soil release polymer and a polysaccharide-based cationic polymer. The anionic polyester soil release polymer and the polysaccharide-based cationic polymer are described below:

<u>The anionic soil release polymer:</u>

The anionic polyester soil release polymer comprises:

A) one or more structural units of the formula (I)

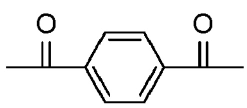

(I)

and
B) one or more structural units of the formula (II)

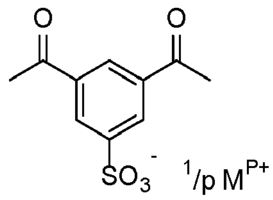

(II)

wherein

$^{1}/_{p}\ M^{p+}$ is a cation, preferably selected from the group consisting of monovalent cations $M^+$ (p = 1), divalent cations ½ $M^{2+}$ (p = 2) and trivalent cations $^{1}/_{3}$ $M^{3+}$ (p = 3) and more preferably selected from the group consisting of $H^+$, $Li^+$, $Na^+$, $K^+$, ½ $Mg^{2+}$, ½ $Ca^{2+}$, $^{1}/_{3}\ Al^{3+}$, $NH_4^+$ and $R^aR^bR^cR^dN^+$, wherein $R^a$, $R^b$, $R^c$ and $R^d$, independently of one another, are H, linear or branched, preferably linear, $(C_1\text{-}C_{22})$-alkyl groups or linear or branched, preferably linear, $(C_2\text{-}C_{10})$-hydroxyalkyl groups, and wherein in the cations $R^aR^bR^cR^dN^+$ at least one of $R^a$, $R^b$, $R^c$ and $R^d$ is not H, and

C) one or more structural units of the formula (III)

$$-O-R^1-O- \qquad (III)$$

wherein

$R^1$ is a linear or branched alkylene group represented by the formula $(C_mH_{2m})$ wherein m is an integer from 2 to 12, preferably from 2 to 6, more preferably from 2 to 4 and even more preferably from 2 to 3, most preferably 3.

and

D) one or more terminal groups of the formula (IV)

$$-O-[C_nH_{2n}-O]_x-R^2 \qquad (IV)$$

wherein

$R^2$ is a linear or branched $C_1\text{-}C_{30}$ alkyl group, a cycloalkyl group with 5 to 9 carbon atoms or a $C_6\text{-}C_{30}$ arylalkyl group, preferably a linear or branched $C_1\text{-}C_{30}$ alkyl group, more preferably a linear $C_1\text{-}C_6$ alkyl group and even more preferably $CH_3$,

n is 2 or an integer > 2, preferably is an integer from 2 to 12, more preferably is an integer from 2 to 6 and even more preferably is an integer from 2 to 4, whereby the definition of n may vary within a single terminal group of the formula (IV), and

x is, based on molar average, a number of at least 30, preferably from 30 to 200, more preferably from 40 to 180, even more preferably from 50 to 150, particularly preferably from 60 to 120 and extraordinarily preferably from 65 to 115.

The one or more structural units of the formula (I) of the polyesters of the invention preferably are derived from terephthalic acid and/or a derivative thereof. Herein, the term "derivative thereof" comprises, but is not limited to, salts thereof, esters thereof, anhydrides thereof, and any mixtures of the foregoing.

More preferably, the one or more structural units of the formula (I) of the polyesters of the invention are derived from terephthalic acid or its dialkyl esters, preferably its $(C_1\text{-}C_4)$-dialkyl esters and more preferably its dimethyl ester.

The one or more structural units of the formula (II) of the polyesters of the invention preferably are derived from 5-sulfoisophthalic acid and/or a derivative thereof. Herein, the term "derivative thereof" comprises, but is not limited to, salts thereof, esters thereof, anhydrides thereof, and any mixtures of the foregoing.

Among "5-sulfoisophthalic acid and/or a derivative thereof" 5-sulfoisophthalic acid sodium salt and dimethyl-5-sulfoisophthalate sodium salt (5-SIM) are preferred.

In the case that one molecule of the polyesters of the invention comprises two or more of the structural units of the formula (II), the definition of $^{1}/_{p}\ M^{p+}$ may vary between those structural units.

The amount of the one or more structural units of the formula (II) in the polyesters of the invention is, on average, preferably from 1 to 80 mol-%, more preferably from 2 to 60 mol-%, even more preferably from 5 to 50 mol-%, particularly preferably from 10 to 40 mol-%, and extraordinarily preferably from 15 to 30 mol-%, in each case based on the combined amount of the one or more structural units of the formula (I) and the one or more structural units of the formula (II) in the polyesters of the invention.

Preferably, the total number of the one or more structural units of the formula (I) and the one or more structural units of the formula (II) in the polyesters of the invention is, based on molar average, from 2 to 30, more preferably from 3 to 22, even more preferably from 4 to 16 and particularly preferably from 5 to 14, such as 7, 9, 12.

The one or more structural units of the formula (III) preferably are derived from alkylene glycol of the formula HO-$R^1$-OH wherein $R^1$ has the meaning given above for formula (III). Preferably, the alkylene glycol is selected from $C_2$-$C_{12}$ alkylene glycol, more preferably from $C_2$-$C_6$ alkylene glycol, even more preferably from $C_2$-$C_4$ alkylene glycol and particularly preferably from $C_2$-$C_3$ alkylene glycol.

In the case that one molecule of the polyesters of the invention comprises two or more of the structural units of the formula (III), the definition of $R^1$ may vary between those structural units.

When the alkylene glycol contains three or more carbon atoms, it is the intention of the invention to cover all possible isomers of the alkylene glycol.

For example, when the alkylene glycol contains three carbon atoms, it can include:

$HO\text{-}CH_2\text{-}CH_2\text{-}CH_2\text{-}OH$,

$HO\text{-}CH_2\text{-}CH(CH_3)\text{-}OH$,

When the alkylene glycol contains 4 carbon atoms, it can include:

$HO\text{-}CH_2\text{-}CH_2\text{-}CH_2\text{-}CH_2\text{-}OH$,

$HO\text{-}CH_2\text{-}CH_2\text{-}CH(CH_3)\text{-}OH$,

$HO\text{-}CH_2\text{-}CH(CH_3)\text{-}CH_2\text{-}OH$,

$HO\text{-}CH(CH_3)\text{-}CH(CH_3)\text{-}OH$.

When the alkylene glycol contains three or more carbon atoms, it is also the intention of the invention to cover all possible ways in which the alkylene glycol may connect with other structural units of the polyester of the invention. For example, when the alkylene glycol is

$HO\text{-}CH_2\text{-}CH(CH_3)\text{-}OH$

The monomer has two possible ways to connect with other structural units of the polyester of the invention:

$\text{-}O\text{-}CH_2\text{-}CH(CH_3)\text{-}O\text{-}$,

or

$\text{-}O\text{-}CH(CH_3)\text{-}CH_2\text{-}O\text{-}$.

Among $C_2$-$C_4$ alkylene glycol ethylene glycol, 1,3-propylene glycol, 1,2-propylene glycol, 1,4-butylene glycol, 1,3-butylene glycol, 2,3-butylene glycol and mixtures thereof are preferred.

Preferably, the polyester of the invention comprises one or more structural units of the formula (III) wherein m is 3.

More preferably, the polyester of the invention comprises one or more structural units of the formula (III) wherein m is 2 and one or more structural units of the formula (III) wherein m is 3.

The one or more structural units of the formula (III) wherein m is 2, preferably are derived from ethylene glycol.

The one or more structural units of the formula (III) wherein m is 3, preferably are derived from 1,2-propylene glycol.

Even more preferably, the one or more structural units of the formula (III) are selected from the group consisting of structural units derived from ethylene glycol, structural units derived from 1,2-propylene glycol and structural units derived from mixtures of ethylene glycol and 1,2-propylene glycol, particularly preferably, the one or more structural units of the formula (III) are selected from the group consisting of structural units derived from 1,2-propylene glycol and structural units

derived from mixtures of ethylene glycol and 1,2-propylene glycol, and extraordinarily preferably, the one or more structural units of the formula (III) are structural units derived from mixtures of ethylene glycol and 1,2-propylene glycol.

In the following, specific examples of structural units of the formula (III) derived from alkylene glycol are given. The structural units of the formula (III) derived from 1,2-propylene glycol have the formula (III-1)

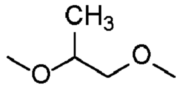
and / or
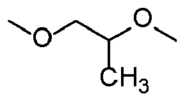

(III-1)

and the structural units of the formula (III) derived from ethylene glycol have the formula (III-2)

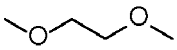

(III-2)

In case the polyesters of the invention comprise one or more structural units of the formula (III-1) and one or more structural units of the formula (III-2), the amount of the one or more structural units of the formula (III-1) in the polyesters of the invention is, on average, preferably from 1 to 100 mol-%, more preferably from 10 to 90 mol-%, even more preferably from 20 to 80 mol-%, particularly preferably from 30 to 70 mol-%, and extraordinarily preferably from 40 to 60 mol-%, in each case based on the combined amount of the one or more structural units of the formula (III-1) and the one or more structural units of the formula (III-2) in the polyesters of the invention.

In the case that one molecule of the polyesters of the invention comprises two or more of the terminal groups of the formula (IV), the definitions of n, x and $R^2$ may vary between those terminal groups.

The one or more terminal groups of the formula (IV) preferably are derived from substances of the formula $HO-[C_nH_{2n}-O]_x-R^2$, wherein n, x and $R^2$ have the meanings given above for formula (IV).

Preferably, x in the one or more terminal groups of the formula (IV) is, based on molar average, a number of at least 50, more preferably from 50 to 200, even more preferably from 50 to 180, particularly preferably from 55 to 150, extraordinarily preferably from 62 to 120 and especially preferably from 67 to 115.

Preferably, n in the one or more terminal groups of the formula (IV) is 2.

Preferably, the one or more terminal groups of the formula (IV) of the polyester of the invention are selected from the formula (IV-a)

$$-O-[C_2H_4-O]_a-[C_3H_6-O]_b-[C_4H_8-O]_c-R^2 \qquad \text{(IV-a)}$$

wherein

$R^2$ is a linear or branched $C_1$-$C_{30}$ alkyl group, a cycloalkyl group with 5 to 9 carbon atoms or a $C_6$-$C_{30}$ arylalkyl group, preferably a linear or branched $C_1$-$C_{30}$ alkyl group, more preferably a linear $C_1$-$C_6$ alkyl group and even more preferably $CH_3$,

a, b and c are, based on molar average, independently of one another, numbers from 0 to 200, the sum of a+b+c is a number of at least 30, preferably from 30 to 200, more preferably from 40 to 180, even more preferably from 50 to 150, particularly preferably from 60 to 120 and extraordinarily preferably from 65 to 115, the $[C_2H_4-O]$, $[C_3H_6-O]$ and/or $[C_4H_8-O]$ units of the one or more terminal groups of the formula (IV-a) may be arranged blockwise, alternating, periodically and/or statistically, preferably blockwise and/or statistically, and either of the $[C_2H_4-O]$, $[C_3H_6-O]$ and $[C_4H_8-O]$ units of the one or more terminal groups of the formula (IV-a) can be linked to $-R^2$ and/or -O.

Any of the units $[C_4H_8-O]$, $[C_3H_6-O]$ and $[C_2H_4-O]$ can be linked to $R^2$- and -O. This means, for example, that both $R^2$- and -O may be connected to a $[C_4H_8-O]$-group, they may both be connected to a $[C_3H_6-O]$-group, they may both be connected to a $[C_2H_4-O]$-group or they may be connected to different groups selected from $[C_4H_8-O]$, $[C_3H_6-O]$ and $[C_2H_4-O]$.

In the case that one molecule of the polyesters of the invention comprises two or more of the terminal groups of the formula (IV-a), the definitions of $R^2$, a, b and c, and the sum of a+b+c may vary between those terminal groups.

The one or more terminal groups of the formula (IV-a) are preferably derived from substances of the formula

$HO-[C_2H_4-O]_a-[C_3H_6-O]_b-[C_4H_8-O]_c-R^2$,

wherein $R^2$, a, b and c, and the sum of a+b+c have the meanings given above for formula (IV-a).

In the one or more terminal groups of the formula (IV-a), the sum of a+b+c preferably is a number of at least 50, more preferably from 50 to 200, even more preferably from 50 to 180, particularly preferably from 55 to 150, extraordinarily preferably from 62 to 120, and especially preferably from 67 to 115.

Preferably, "a" in the one or more terminal groups of the formula (IV-a) is, based on molar average, a number from 30 to 200, more preferably from 40 to 180, even more preferably from 50 to 150, particularly preferably from 60 to 120 and extraordinarily preferably from 65 to 115.

More preferably, "a" in the one or more terminal groups of the formula (IV-a) is, based on molar average, a number from 50 to 200, even more preferably from 50 to 180, particularly preferably from 55 to 150, extraordinarily preferably from 62 to 120, and especially preferably from 67 to 115.

Preferably, "b" in the one or more terminal groups of the formula (IV-a) is, based on molar average, a number from 0 to 50, more preferably from 0 to 20, even more preferably from 0 to 10 and particularly preferably "b" is 0.

Preferably, "c" in the one or more terminal groups of the formula (IV-a) is 0.

More preferably, "b" and "c" in the one or more terminal groups of the formula (IV-a) are 0.

Even more preferably, in the one or more terminal groups of the formula (IV-a)

$R^2$ is a linear or branched $C_1$-$C_{30}$ alkyl group, a cycloalkyl group with 5 to 9 carbon atoms or a $C_6$-$C_{30}$ arylalkyl group, preferably a linear or branched $C_1$-$C_{30}$ alkyl group, more preferably a linear $C_1$-$C_6$ alkyl group and even more preferably $CH_3$,

b and c are both 0, and

a is, based on molar average, a number of from 30 to 200, preferably from 40 to 180, more preferably from 50 to 150, even more preferably from 60 to 120 and particularly preferably from 65 to 115.

In a particularly preferred embodiment of the invention, in the one or more terminal groups of the formula (IV-a)

$R^2$ is a linear or branched $C_1$-$C_{30}$ alkyl group, a cycloalkyl group with 5 to 9 carbon atoms or a $C_6$-$C_{30}$ arylalkyl group, preferably a linear or branched $C_1$-$C_{30}$ alkyl group, more preferably a linear $C_1$-$C_6$ alkyl group, and even more preferably $CH_3$,

b and c are both 0, and

a is, based on molar average, a number from 50 to 200, preferably from 50 to 180, more preferably from 55 to 150, even more preferably from 62 to 120 and particularly preferably from 67 to 115.

Even more preferably, in the one or more terminal groups of the formula (IV-a), $R^2$ is $CH_3$, b and c are 0 and a is, based on molar average, a number selected from the group consisting of 33, 40, 45, 56, 67, 79, 90, 102 and 113.

Examples of the one or more terminal groups of the formula (IV) or (IV-a) are terminal groups derived from polyethylene glycol) monomethyl ether (mPEG), preferably terminal groups derived from mPEG selected from the group consisting of mPEG1500, mPEG1800, mPEG2000, mPEG2500, mPEG3000, mPEG3500, mPEG4000, mPEG4500 and mPEG5000 and more preferably terminal groups derived from mPEG selected from the group consisting of mPEG3000 and mPEG4000.

The number in the terms beginning with "mPEG" from the previous paragraph describes the average molecular weight of the polyethylene glycol) monomethyl ether in g/mol.

In a preferred embodiment of the invention, the polyester of the invention, which is hereinafter referred to as "polyester A" comprises, and preferably consists of one or more structural units of the formula (I), and one or more structural units of the formula (II) wherein $^1/_p\ M^{p+}$ has the meaning given above, and one or more structural units of the formula (III) and preferably one or more structural units of the formula (III), and one or more terminal groups of the formula (IV-a)

wherein

$R^2$ is a linear or branched $C_1$-$C_{30}$ alkyl group, a cycloalkyl group with 5 to 9 carbon atoms or a $C_6$-$C_{30}$ arylalkyl group, preferably a linear or branched $C_1$-$C_{30}$ alkyl group, more preferably a linear $C_1$-$C_6$ alkyl group and even more preferably $CH_3$, and

a, b and c are, based on molar average, independently of one another, numbers from 0 to 200, the sum of a+b+c is a number of at least 30, preferably from 30 to 200, more preferably from 40 to 180, even more preferably from 50 to 150, particularly preferably from 60 to 120 and extraordinarily preferably from 65 to 115, the

$[C_2H_4\text{-}O]$, $[C_3H_6\text{-}O]$ and/or $[C_4H_8\text{-}O]$ units of the one or more terminal groups of the formula (IV-a) may be arranged blockwise, alternating, periodically and/or statistically, preferably blockwise and/or statistically, and either of the $[C_2H_4\text{-}O]$, $[C_3H_6\text{-}O]$ and $[C_4H_8\text{-}O]$ units of the one or more terminal groups of the formula (IV-a) can be linked to $-R^2$ and/or -O.

Preferably, in the "polyester A"

a, b and c are, based on molar average, independently of one another, numbers from 0 to 200, the sum of a+b+c is a number of at least 50, preferably from 50 to 200, more preferably from 50 to 180, even more preferably from 55 to 150, particularly preferably from 62 to 120 and extraordinarily preferably from 67 to 115, the $[C_2H_4\text{-}O]$, $[C_3H_6\text{-}O]$ and/or $[C_4H_8\text{-}O]$ units of the one or more terminal groups of the formula (IV-a) may be arranged blockwise, alternating, periodically and/or statistically, preferably blockwise and/or statistically, and either of the $[C_2H_4\text{-}O]$, $[C_3H_6\text{-}O]$ and $[C_4H_8\text{-}O]$ units of the one or more terminal groups of the formula (IV-a) can be linked to $-R^2$ and/or -O.

In a preferred embodiment of the invention, the polyester of the invention further comprises one or more additional structural units of the formula (V)

$$-O-[C_{n1}H_{2n1}-O]_d- \qquad (V)$$

wherein

n1 is 2 or an integer > 2, preferably is an integer from 2 to 12, more preferably is an integer from 2 to 6 and even more preferably is an integer from 2 to 4,

d is, based on molar average, a number from 2 to 200, preferably from 3 to 100, more preferably from 4 to 50, and even more preferably from 5 to 25,

and whereby the definition of n1 may vary within a single structural unit of the formula (V), and the average number of moles of the one or more structural units of the formula (V) per mole of the polyester preferably is 0.3 or more than 0.3.

n the case that one molecule of the polyesters of the invention comprises two or more of the structural units of the formula (V), the definitions of n1 and d may vary between those structural units.

Herein, the structural units of the formula (V) are specifically defined to be different versus structural unit of the formula (IV). The one or more structural units of the formula (V) are derived from polyalkyleneglycol of the formula $HO-[C_{n1}H_{2n1}-O]_d-H$, wherein n1 and d have the meanings given above for formula (V). In formula $HO-[C_{n1}H_{2n1}-O]_d-H$, both OH groups at the two ends are open to form esters, this is different versus structural (IV) where only one OH group is open to form esters, the other OH is connected to $R^2$ and not open to form esters.

The term "polyalkyleneglycol" includes the homopolymers of alkylene oxide (including but not limited to ethylene oxide (EO), propylene oxide (PO) and/or butylene oxide (BO)); or the copolymers of alkylene oxide (including but not limited to ethylene oxide, propylene oxide and/or butylene oxide). When the polyalkyleneglycol is a copolymer, the different types of alkylene oxide may be arranged blockwise, alternating, periodically and/or statistically. Preferably, the polyalkyleneglycol is a homopolymer, preferably a homopolymer of ethylene oxide, or a block copolymer. Preferred polyalkyleneglycol block copolymers are EO/PO diblock, EO/PO/EO tri-block, PO/EO/PO tri-block.

Preferably, the one or more structural units of the formula (V) are selected from the formula (V-a)

$$-O-[C_2H_4-O]_d- \qquad (V\text{-}a)$$

wherein d is, based on molar average, a number from 2 to 200, preferably from 3 to 100, more preferably from 4 to 50, and even more preferably from 5 to 25, and the average number of moles of the one or more structural units of the formula (V-a) per mole of the polyester preferably is 0.3 or more than 0.3.

In the case that one molecule of the polyester of the invention comprises two or more of the structural units of the formula (V-a), the definition of d may vary between those structural units.

The one or more structural units of the formula (V-a) preferably are derived from polyethylene glycol of the formula $HO-[C_2H_4-O]_d-H$, wherein d has the meaning given above for formula (V-a).

Particularly preferably, in the one or more structural units of the formula (V-a), d is, based on molar average, a number selected from the group consisting of 4, 6, 9, 11, 22, 34, 45, 56, 68, 79 and 91.

Examples of the one or more structural units of the formula (V) or (V-a) are structural units derived from poly(ethylene glycol) (PEG) and preferably are structural units derived from PEG selected from the group consisting of PEG200, PEG300, PEG400, PEG500, PEG1000, PEG1500, PEG2000, PEG2500, PEG3000, PEG3500 and PEG4000.

The number in the terms beginning with "PEG" from the previous paragraph describes the average molecular weight of the polyethylene glycol) in g/mol.

The average number of moles of the one or more structural units of the formula (V), preferably selected from the structural units of the formula (V-a), per mole of the polyester of the invention, preferably is 0.3 or more than 0.3, more preferably is 0.5 or more than 0.5, even more preferably is 0.7 or more than 0.7, particularly preferably is 1 or more than 1 and extraordinarily preferably is 1.

When calculating the average number of moles of the one or more structural units of the formula (V), preferably selected from the structural units of the formula (V-a), per mole of the polyester of the invention, only structural units different from structural units derived from mono alkylene glycols are considered.

In the polyesters of the invention, the one or more structural units of the formula (V) and the one or more structural units of the formula (V-a) are not linked directly to a linear or branched $C_1$-$C_{30}$ alkyl group, a cycloalkyl group with 5 to 9 carbon atoms or a $C_6$-$C_{30}$ arylalkyl group.

In a further preferred embodiment of the invention, the polyester of the invention comprises one or more structural units which are derived from dicarboxylic acids and/or derivatives thereof and different from the one or more structural units of the formulae (I) and (II). In case the polyester of the invention comprises such one or more structural units which are derived from dicarboxylic acids and/or derivatives thereof and different from the one or more structural units of the formulae (I) and (II), these structural units preferably are derived from substances selected from the group consisting of phthalic acid, isophthalic acid, 3-sulfophtahlic acid, 4-sulfophtahlic acid, naphthalene-1,4-dicarboxylic acid, naphthalene-2,6-dicarboxylic acid, tetrahydrophthalic acid, diphenoxyethane-4,4'-dicarboxylic acid, diphenyl-4,4'-dicarboxylic acid, 2,5-furandicarboxylic acid, adipic acid, sebacic acid, decan-1,10-dicarboxylic acid, fumaric acid, succinic acid, 1,4-cyclohexanedicarboxylic acid, cyclohexanediacetic acid, glutaric acid, azelaic acid, and/or derivatives thereof and mixtures thereof. Herein, the term "derivative thereof" comprises, but is not limited to, salts thereof, esters thereof, anhydrides thereof, and any mixtures of the foregoing. In case the aforementioned one or more structural units which are derived from dicarboxylic acids and/or derivatives thereof and different from the one or more structural units of the formulae (I) and (II) comprise a sulfo group, this sulfo group is of the formula $-SO_3^-\ ^1/_p\,M^{p+}$, wherein the cation $^1/_p\,M^{p+}$ preferably has the meaning given above, and more preferably is $Na^+$.

Typically, such one or more structural units which are derived from dicarboxylic acids and/or derivatives thereof and different from the one or more structural units of the formulae (I) and (II) would be present to a minor extent, preferably in an amount smaller than 5 wt.-%, based on the total weight of the polyester of the invention.

In case the polyester of the invention comprises one or more structural units which are derived from dicarboxylic acids and/or derivatives thereof and different from the one or more structural units of the formulae (I) and (II), these structural units are preferably derived from substances selected from the group consisting of isophthalic acid, 1,4-cyclohexanedicarboxylic acid, 2,5-furandicarboxylic acid, derivatives thereof and mixtures of the aforementioned.

In a further preferred embodiment of the invention, the polyester of the invention comprises one or more anionic terminal groups of the formulae

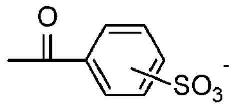 $^1/_p\,M^{P+}$

or $-O-[C_2H_4O]_t-SO_3^-\ ^1/_p\,M^{p+}$ wherein,

$^1/_p\,M^{p+}$ is a cation, preferably selected from the group consisting of monovalent cations $M^+$ (p = 1), divalent cations ½ $M^{2+}$ (p = 2) and trivalent cations $^1/_3\,M^{3+}\ (p = 3)$ and more preferably selected from the group consisting of $H^+$, $Li^+$, $Na^+$, $K^+$, ½ $Mg^{2+}$, ½ $Ca^{2+}$, $^1/_3\,Al^{3+}$ $Al^{3+}$, $NH_4^+$ and $R^aR^bR^cR^dN^+$, wherein $R^a$, $R^b$, $R^c$ and $R^d$, independently of one another, are H, linear or branched, preferably linear, ($C_1$-$C_{22}$)-alkyl groups or linear or branched, preferably linear, ($C_2$-$C_{10}$)-hydroxyalkyl groups, and wherein in the cations $R^aR^bR^cR^dN^+$ at least one of $R^a$, $R^b$, $R^c$ and $R^d$ is not H, and

t is, based on molar average, a number from 1 to 10, preferably from 1 to 4, and more preferably t is 1.

n a further preferred embodiment of the invention, the polyester of the invention comprises crosslinking structural units derived from one or more crosslinking agents.

Herein, the crosslinking agent is defined as an organic molecule which comprises three or more functional groups selected from carboxylic acid group; salts, esters, or anhydrides of carboxylic acid (whereby an anhydride group of carboxylic acids is equivalent to two carboxylic acid groups); hydroxyl group; and any mixture thereof. Examples of

crosslinking agents comprise, but are not limited to, citric acid (contains 3 carboxylic acid groups and 1 hydroxyl group), trimellitic acid (contains 3 carboxylic acid groups), glycerol (contains 3 hydroxyl groups), and sugar alcohols such as sorbitol, mannitol, erythritol, etc.

Typically, such crosslinking structural units would be present to a minor extent, preferably in an amount smaller than 5 wt.-%, more preferably in an amount smaller than 3 wt.-%, and even more preferably in an amount smaller than 1 wt.-%, in each case based on the total weight of the polyester of the invention.

Preferably, in the polyester of the invention, the amount of the one or more terminal groups of the formula (IV), preferably selected from the terminal groups of the formula (IV-a), is, in each case based on the total weight of the polyester, at least 40 wt.-%, more preferably at least 50 wt.-% and even more preferably at least 60 wt.-%.

Preferably, in the polyester of the invention, the combined amount of the one or more structural units of the formula (I), and the one or more structural units of the formula (II), and the one or more structural units of the formula (III), and the one or more terminal groups of the formula (IV), preferably selected from the terminal groups of the formula (IV-a), and, if present, the one or more structural units of the formula (V), preferably selected from the structural units of the formula (V-a), and, if present, the one or more structural units derived from mono alkylene glycol different from 1,2-propylene glycol, is at least 50 wt.-%, more preferably is at least 60 wt.-% and even more preferably is at least 70 wt.-%, in each case based on the total weight of the polyester.

In a preferred embodiment of the invention, the polyester of the invention consists exclusively of the one or more structural units of the formula (I), and of the one or more structural units of the formula (II), and of the one or more structural units of the formula (III), and of the one or more terminal groups of the formula (IV), preferably selected from the terminal groups of the formula (IV-a), and, if present, of the one or more structural units of the formula (V), preferably selected from the structural units of the formula (V-a), and, if present, of the one or more structural units derived from mono alkylene glycol different from 1,2-propylene glycol.

In a more preferred embodiment of the invention, the polyester of the invention consists exclusively of the one or more structural units of the formula (I), and of the one or more structural units of the formula (II), and of the one or more structural units of the formula (III), and of the one or more terminal groups of the formula (IV), preferably selected from the terminal groups of the formula (IV-a).

In another more preferred embodiment of the invention, the polyester of the invention consists exclusively of the one or more structural units of the formula (I), and of the one or more structural units of the formula (II), and of the one or more structural units of the formula (III), and of the one or more terminal groups of the formula (IV), preferably selected from the terminal groups of the formula (IV-a), and of the one or more structural units of the formula (V), preferably selected from the structural units of the formula (V-a).

In another more preferred embodiment of the invention, the polyester of the invention consists exclusively of the one or more structural units of the formula (I), and of the one or more structural units of the formula (II), and of the one or more structural units of the formula (III), and of the one or more terminal groups of the formula (IV), preferably selected from the terminal groups of the formula (IV-a), and of the one or more structural units derived from mono alkylene glycol different from 1,2-propylene glycol.

In another more preferred embodiment of the invention, the polyester of the invention consists exclusively of the one or more structural units of the formula (I), and of the one or more structural units of the formula (II), and of the one or more structural units of the formula (III), and of the one or more terminal groups of the formula (IV), preferably selected from the terminal groups of the formula (IV-a), and of the one or more structural units of the formula (V), preferably selected from the structural units of the formula (V-a), and of the one or more structural units derived from mono alkylene glycol different from 1,2-propylene glycol.

When no cross-linking agent is used for the preparation of the polyesters of the invention, polyesters are formed which possess a linear structure and contain a terminal group of the formula (IV) at one end of the polyester or a terminal group of the formula (IV) at both ends of the polyester. Preferably, the polyester of the invention possesses a linear structure, i. e. does not comprise cross-linking structures, and contains a terminal group of the formula (IV) at both ends of the polyester. When a cross-linking agent is used for the preparation of the polyesters of the invention, the respective polyesters may comprise more than 2 terminal groups of the formula (IV).

In case the polyester of the invention contains only one terminal group of the formula (IV), the polyester of the invention comprises one or more further terminal groups different from the terminal group of the formula (IV). These terminal groups may result from other reactants used for the preparation of the polyester. Preferably, these terminal groups are selected from the group consisting of -OH, $-OCH_3$ (these two terminal groups can e. g. occur in case a structural unit of the formula (I) or (II) terminates an end of the polyester), $-O-CH(CH_3)-CH_2-OH$, $-O-CH_2-CH(CH_3)-OH$ (these terminal groups can e. g. occur in case a structural unit of the formula (III) terminates an end of the polyester), $-O-[C_{n1}H_{2n1}-O]_dH$, wherein n1 and d have the meanings given above for formula (V) and whereby the definition of n1 may vary within a single terminal group (this terminal group can e. g. occur in case a structural unit of the formula (V) terminates an end of the polyester).

In a further preferred embodiment of the invention, the polyester of the invention has the formula (X)

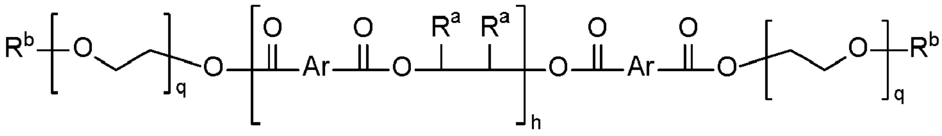

(X)

wherein

$R^a$ is, each independently, selected from the group consisting of H and $CH_3$, whereby the polyester comprises one or more structural units -O-CHR$^a$-CHR$^a$-O- wherein one of the two residues $R^a$ is H and the other of the two residues $R^a$ is $CH_3$, preferably, the one or more structural units -O-CHR$^a$-CHR$^a$-O- are selected from the group consisting of -O-$CH_2$-$CH_2$-O-, -O-$CH_2$-CH($CH_3$)-O-, -O-CH($CH_3$)-$CH_2$-O- and mixtures thereof, whereby the polyester comprises one or more structural units -O-CHR$^a$-CHR$^a$-O- wherein one of the two residues $R^a$ is H and the other of the two residues $R^a$ is $CH_3$, and more preferably, the one or more structural units-O-CHR$^a$-CHR$^a$-O- are mixtures of one or more structural units -O-$CH_2$-$CH_2$-O-and one or more structural units -O-CHR$^a$-CHR$^a$-O- wherein one of the two residues $R^a$ is H and the other of the two residues $R^a$ is $CH_3$,

$R^b$ is, each independently, a linear $C_1$-$C_6$ alkyl group, more preferably $CH_3$,

q is, based on molar average, each independently, a number of at least 30, preferably from 30 to 200, more preferably from 40 to 180, even more preferably from 50 to 150, particularly preferably from 60 to 120 and extraordinarily preferably from 65 to 115,

Ar represents, each independently

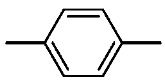

or

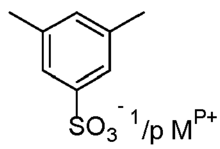

(X-1) (X-2)

the polyester comprising both, one or more structural units of the formula (X-1) and one or more structural units of the formula (X-2),

$^1/_p M^{p+}$ $M^{p+}$ is a cation, preferably selected from the group consisting of monovalent cations $M^+$ (p = 1), divalent cations ½ $M^{2+}$ (p = 2) and trivalent cations $^1/_3 M^{3+}$ (p = 3) and more preferably selected from the group consisting of $H^+$, $Li^+$, $Na^+$, $K^+$, ½ $Mg^{2+}$, ½ $Ca^{2+}$, $^1/_3 Al^{3+}$, $Al^{3+}$, $NH_4^+$ and $R^aR^bR^cR^dN^+$, wherein $R^a$, $R^b$, $R^c$ and $R^d$, independently of one another, are H, linear or branched, preferably linear, ($C_1$-$C_{22}$)-alkyl groups or linear or branched, preferably linear, ($C_2$-$C_{10}$)-hydroxyalkyl groups, and wherein in the cations $R^aR^bR^cR^dN^+$ at least one of $R^a$, $R^b$, $R^c$ and $R^d$ is not H, and

h is, based on molar average, a number from 1 to 29, preferably from 2 to 21, more preferably from 4 to 15 and even more preferably from 5 to 13.

In a preferred embodiment of the invention, "q" in the inventive polyesters of the formula (X) is, based on molar average, each independently, a number of at least 50, more preferably from 50 to 200, even more preferably from 50 to 180, particularly preferably from 55 to 150, extraordinarily preferably from 62 to 120 and especially preferably from 67 to 115.

Preferably, the polyesters of the invention are biodegradable. The biodegradability of polyesters is determined following the OECD 301B Ready Biodegradability $CO_2$ Evolution Test Guideline. In this test, the test substance is the sole carbon and energy source and under aerobic conditions microorganisms metabolize the test substance producing $CO_2$ or incorporating the carbon into biomass. The amount of $CO_2$ produced by the test substance (corrected for the $CO_2$ evolved by the blank inoculum) is expressed as a percentage of the theoretical amount of $CO_2$ ($ThCO_2$) that could have been produced if the organic carbon in the test substance was completely converted to $CO_2$. The polyesters of the present invention show biodegradability of more than 40%, preferably more than 50%, more preferably more than 60% within 60 days, preferably within 28 days.

It is to be understood that the polyesters of the invention are typically prepared by polycondensation processes. This leads to statistically determined mixtures of polyesters in which a mixture of molecular species with a distribution around a molar average is obtained. Furthermore, small amounts of polyester may be present within the statistically determined mixtures of polyesters which do not comprise structural units of the formula (I) or (II).

Preferably, the weight average molecular weight (MW) of the polyester of the invention is from 2000 to 20000 g/mol and more preferably from 3000 to 18000 g/mol.

The weight average molecular weight (MW) of the polyesters of the invention may be determined by gel permeation chromatography (GPC) analysis, preferably as detailed in the following: 20 µl of sample with a concentration of 1 mg/ml dissolved in tetrahydrofuran (THF) / $H_2O$ 80:20 (v:v) is injected onto a PSS Suprema column set of two columns with the dimensions 300 mm length and 8 mm internal diameter (ID) with a porosity of 30 Å and particle size 10 µm. The detection is monitored at 235 nm on a multiple wavelength detector. The employed eluent is 1.25 g/l of disodium hydrogen phosphate dihydrate in a 45 / 55 % (v/v) water / acetonitrile mixture. Separations are conducted at a flowrate of 1 ml/minute and 25 °C. Quantification is performed by externally calibrating standard samples of different molecular weight polyethylene glycols (430 g/mol - 44000 g/mol). The used SEC columns are consisting of a modified acrylate copolymer network.

The groups ($C_2H_4$) in the terminal groups of the formula (IV-a) and the structural units of the formula (V-a) preferably are of the formula $-CH_2-CH_2-$. The same applies in case the structural units of the formula (V) or the terminal groups of the formula (IV) comprise one or more groups ($C_2H_4$).

The groups ($C_3H_6$) in the terminal groups of the formula (IV-a) preferably are of the formula $-CH(CH_3)-CH_2-$ or $-CH_2-CH(CH_3)-$, i. e. of the formula:

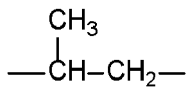

or

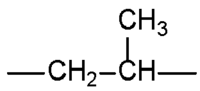

The same applies in case the structural units of the formula (V) or the terminal groups of the formula (IV) comprise one or more groups ($C_3H_6$).

The groups ($C_4H_8$) in the terminal groups of the formula (IV-a) preferably are of the formula $-CH(CH_3)-CH(CH_3)-$, i. e. of the formula

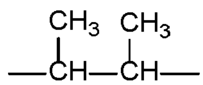

The same applies in case the structural units of the formula (V) or the terminal groups of the formula (IV) comprise one or more groups ($C_4H_8$).

In the polyesters of the invention, the structural units or terminal groups of the formula (III), (IV), (IV-a), (V), or (V-a) generally are linked directly to structural units of the formula (I) or (II). Ester groups result. However, in the polyesters of the invention, the structural units or terminal groups of the formula (III), (IV), (IV-a), (V), or (V-a) generally are not linked directly to other structural units or terminal groups of the formula (III), (IV), (IV-a), (V), or (V-a). Likewise, in the polyesters of the invention, the structural units of the formula (I) or (II) generally are not linked directly to other structural units of the formula (I) or (II).

For the preparation of the polyesters of the invention, typically a two-stage process is used of either direct esterification of dicarboxylic acids and diols or transesterification of (i) diesters of dicarboxylic acids and (ii) diols, followed by a polycondensation reaction under reduced pressure.

A further subject matter of the invention is a process for the preparation of the polyesters of the invention, comprising the steps of heating terephthalic acid and/or a derivative thereof, preferably dimethyl terephthalate, and 5-sulfoisophthalic acid and/or a derivative thereof, preferably dimethyl-5-sulfoisophthalate sodium salt, and 1,2-propylene glycol, and one or more substances of the formula $HO-[C_nH_{2n}-O]_x-R^2$ wherein n, x and $R^2$ have the meanings given above for formula (IV) and whereby the definition of n may vary within a single molecule of the formula $HO-[C_nH_{2n}-O]_x-R^2$, and preferably one or more substances of the formula $HO-[C_2H_4-O]_a-[C_3H_6-O]_b-[C_4H_8-O]_c-R^2$ wherein a, b, c, the sum of a+b+c, and $R^2$ have the meanings given above for formula (IV-a) and whereby the $[C_2H_4-O]$, $[C_3H_6-O]$ and/or $[C_4H_8-O]$ units of the one or more substances of the formula $HO-[C_2H_4-O]_a-[C_3H_6-O]_b-[C_4H_8-O]_c-R^2$ may be arranged blockwise, alternating, periodically and/or statistically, preferably blockwise and/or statistically, and either of the $[C_2H_4-O]$, $[C_3H_6-O]$ and $[C_4H_8-O]$ units of the one or more substances of the formula $HO-[C_2H_4-O]_a-[C_3H_6-O]_b-[C_4H_8-O]_c-R^2$ can be linked to $-R^2$ and/or -OH, and, optionally, one or more substances of the formula $HO-[C_{n1}H_{2n1}-O]_dH$ wherein n1 and d have the meanings given above for formula (V) and whereby the definition of n1 may vary within a single molecule of the formula $HO-[C_{n1}H_{2n1}-O]_dH$, preferably one or more substances of the formula $HO-[C_2H_4-O]_dH$ wherein d has the meaning given above for formula (V-a), and, optionally, one or more mono alkylene glycols different from 1,2-propylene glycol, preferably ethylene glycol, with the addition of a catalyst, to temperatures of 160 to 220°C, preferably beginning at atmospheric pressure, and then continuing the reaction under reduced pressure at temperatures of from 160 to 240°C.

Reduced pressure preferably means a pressure of from 0.1 to 900 mbar and more preferably a pressure of from

0.5 to 500 mbar.

In a preferred embodiment of the process of the invention, individual components or reactants may be added at different times during the reaction process but preferably before the reaction is continued under reduced pressure at temperatures of from 160 to 240°C.

Typical transesterification and condensation catalysts known in the art can be used for the inventive process for the preparation of the polyesters of the invention, such as antimony, germanium and titanium-based catalysts. Preferably, tetraisopropyl orthotitanate (IPT) and sodium acetate (NaOAc) are used as the catalyst system in the inventive process for the preparation of the polyesters of the invention.

The polyesters of the invention may be used in substance, i. e. as granules, but may also be provided as solutions or dispersions. The latter two exhibit beneficial handling properties and are more easily dosed. Preferably, the solutions or dispersions comprise the polyesters of the invention in an amount of from 10 to 80 wt.-% based on the total weight of the solution or dispersion. Suitable solvents for such solutions or dispersions are for example water, ethanol, propanol, butanol, ethylene glycol, 1,2-propylene glycol, 1,3-propylene glycol, 1,2-butylene glycol, 1,3-butylene glycol, 1,4-butylene glycol, butyl glycol, butyl diglycol, butyl polyglycol, glycerol or mixtures thereof. These solvents are preferably used in an amount of from 20 to 90 wt.-%, based on the total weight of the solution or dispersion.

A further subject matter of the invention is solutions or dispersions comprising one or more polyesters of the invention, preferably in an amount of from 10 to 80 wt.-%, based on the total weight of the solution or dispersion, and one or more solvents selected from the group consisting of water, ethanol, propanol, butanol, ethylene glycol, 1,2-propylene glycol, 1,3-propylene glycol, 1,2-butylene glycol, 1,3-butylene glycol, 1,4-butylene glycol, butyl glycol, butyl diglycol, butyl polyglycol, glycerol, and mixtures thereof, preferably in an amount of from 20 to 90 wt.-%, in each case based on the total weight of the solution or dispersion.

In a preferred embodiment of the invention, the solution or dispersion of the invention further comprises one or more polyesters different from the polyesters of the invention, preferably nonionic polyesters, more preferably nonionic polyesters showing a detergency enhancement during laundry applications and even more preferably nonionic soil release polyesters.

The nonionic soil release polymer or polyester can be biodegradable or non-biodegradable, but preferably is biodegradable. Suitable nonionic soil release polyesters include, for example, but are not limited to, Texcare SRN260 or TexCare SRN170 from Clariant.

The solution or dispersion of the invention may be transparent or opaque, white or slightly yellowish. The solution or dispersion of the invention may be used to provide an opaque appearance for the finished product or for a part of the finished product.

The raw materials for the preparation of the polyesters of the invention can be based on fossil carbon or renewable carbon. Renewable carbon includes carbon originating from biomass, carbon capture, or chemical recycling. Preferably, the raw materials for the preparation of the polyesters of the invention are at least partly based on renewable carbon. The Renewable Carbon Index (RCI, a measure of sustainability by dividing the number of carbons derived from renewable sources by the total number of carbons in an active ingredient) of the polyesters of the invention preferably is above 40%, more preferably above 50%, even more preferably above 60%, particularly preferably from 70 to 100%, and most preferably 100%. In a preferred embodiment of the invention, all the $-CH_2-CH_2-O-$ structural units within structural units of the formula (V-a) and terminal groups of the formula (IV-a), as well as all the $-CH_2-CH_2-O-$ structural units within structural units of the formula (V) and terminal groups of the formula (IV), in case these comprise one or more structural units $-CH_2-CH_2-O-$, are bio-based, and the polyesters of the invention have a RCI above 40%, preferably from 50 to 95% and more preferably from 60 to 85%.

The polyesters of the present invention in particular show advantageous performance in laundry detergent compositions, preferably advantageous washing performance in laundry detergent compositions, and possess advantageous biodegradability.

During the use of fabric and home care compositions comprising the polyester of the invention, the polyester can deposit on surfaces, especially fabric surfaces which comprise synthetic fibers, such as polyester, etc. The deposition of the polyester of the invention gives anti-fouling properties to the fabric surfaces: various soil (including body soil, grease soil, clay, biological stains, or microorganisms) have reduced adhesion to the polyester treated fabric surfaces, so that less soil can deposit on these surfaces during wash and wear. Furthermore, when soil is attached to a fabric surface treated with a polyester of the invention, it can be more easily removed in later washing procedures because of reduced adhesion between soil and fabric. Overall, the polyester of the invention can bring various benefits including reduced soil deposition onto the fabric during the washing process and during wear, reduced adhesion of microorganisms and allergens onto the fabric, whiteness maintenance, easier soil removal from fabrics which have been treated with polyester of the invention in a previous washing process, i.e. soil release performance, malodor reduction or control, improved or maintained wicking properties of a fabric, etc.

Furthermore, the polyesters of the invention show advantageous processability and advantageous stability, e. g. in fabric and home care compositions such as laundry detergent compositions.

Typically, the level of polyester in the fabric and home care composition is from about 0.01% to about 10.0 % by weight of the composition, preferably from about 0.05% to about 5%, and more preferably from about 0.1% to about 3.0% by weight of the composition.

The polysaccharide-based cationic polymer:

Polysaccharide-based cationic polymers are derived from chemical modification of a polysaccharide backbone to incorporate overall cationic charge to the polymer. In the present invention, various polysaccharide backbone can be used for chemical modification, including, but not limit to cellulose, starch, glycogen, guar, dextran, polyglucan, chitin, pectins, curdlan, xylose, Inulin, pullulan, locust bean gum, cassia gum, tamarind gum (xyloglucan), xanthan gum, amylose, amylopectin, scleroglucan and mixtures thereof.

Preferably, the polysaccharide backbone described herein have a weight average molecular weight of from 5K to 2000K Daltons, more preferably from 10K to 1000K Daltons, more preferably from 20K to 600K Daltons, most preferably from 30K to 400K.

The cationic modified polysaccharide can be derived from chemical modification of the polysaccharide backbone mentioned above with at least one positively charged organic group. The positively charged organic group typically has at least one hydrogen atom substituted by a positively charged substitution group. The positively charged organic group may be further substituted by other substation groups, such as hydroxyl groups (-OH), acyl group (-CO-R), etc.

The positively charged organic group can be independently connected to the polysaccharide backbone via various linkages, including, but not limited to, an ether (-O-) linkage, or an ester (-O-C=O-) linkage, or a carbamate ( 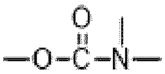 ) linkage.

The positively charged substitution group may be represented by structure below:

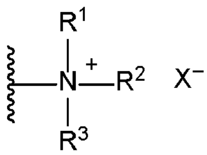

(Structure I)

wherein, $R^1$, $R^2$ and $R^3$ are each independently selected from a H; a linear or branched, substituted or non-substituted $C_1$-$C_{30}$ alkyl group, a substituted or non-substituted $C_5$-$C_9$ cycloalkyl group, a substituted or non-substituted $C_6$-$C_{30}$ arylalkyl group, or a substituted or non-substituted $C_6$-$C_{30}$ alkylaryl group. Preferably, at least one from $R^1$, $R^2$ and $R^3$ is not H. More preferably, at least two from $R^1$, $R^2$ and $R^3$ is not H. Most preferably, $R^1$, $R^2$ and $R^3$ are each independently selected from a linear or branched $C_1$-$C_{20}$ alkyl group.

The $X^-$ represents a counter ion for the positively charged organic group, the counter ion can be any suitable anion, including an acetate, bromide, carbonate, chloride, hydrogen carbonate, hydrogen phosphate, hydrogen sulfate, preferably chloride ($Cl^-$).

A preferred example of positively charged organic group can be represented by the following structure:

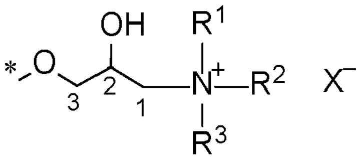

(Structure II)

wherein, each of $R^1$, $R^2$ and $R^3$ are independently selected from a linear or branched, substituted or non-substituted $C_1$ to $C_{18}$ alkyl. Preferably, $R^1$ and $R^2$ are both $CH_3$, and $R^3$ is $C_{12}$-$C_{14}$ alkyl. More preferably, $R^1$, $R^2$ and $R^3$ are all $CH_3$.

The "*" represent a carbon of the polysaccharide backbone. This positively charged organic group of Structure II comprises three carbon atoms ($C_1$, $C_2$ and $C_3$). One hydrogen atom on $C_1$ is substituted by a positively charged substitution group as defined herein. One hydrogen atom on $C_2$ is further substituted by a hydroxyl group (OH). The positively charged organic group is connected to a carbon of the polysaccharide backbone via $C_3$ through an ether linkage (-O-).

The $X^-$ represent a counter ion for the positively charged organic group, and this counter ion can be any suitable anion, including an acetate, bromide, carbonate, chloride, hydrogen carbonate, hydrogen phosphate, hydrogen sulfate, preferably chloride ($Cl^-$).

Another preferred example of positively charged organic group can be represented by the following structure:

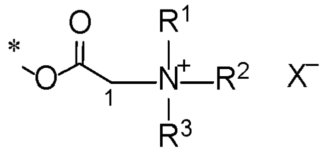

(Structure III)

wherein, each of $R^1$, $R^2$ and $R^3$ are each independently selected from a linear or branched, substituted or non-substituted $C_1$ to $C_{18}$ alkyl. Preferably, both $R^1$ and $R^2$ are $CH_3$, and $R^3$ is $C_{12}$-$C_{14}$ alkyl. More preferably, $R^1$, $R^2$ and $R^3$ are all $CH_3$.

The "*" represent a carbon of the polysaccharide backbone. This specific positively charged organic group (Structure III) comprises one carbon atoms ($C_1$). One hydrogen atom on $C_1$ is substituted by a positively charged substitution group as defined herein. The positively charged organic group is connected to a carbon of the polysaccharide backbone via $C_1$ through an ester linkage (-O-C=O-).

The $X^-$ represents a counter ion for the positively charged organic group, the counter ion can be any suitable anion, including an acetate, bromide, carbonate, chloride, hydrogen carbonate, hydrogen phosphate, hydrogen sulfate, preferably chloride ($Cl^-$).

It is understood that $R^1$, $R^2$ and $R^3$ in above structure (I), (II), (III) can be further substituted. For example, Structure (IV) below can be considered as a specific class under Structure (III) with both $R^1$ and $R^2$ are $CH_3$, $R^3$ is $-CH_2CH_2CH_2$-NH-(CO)-$R^4$, wherein $R^4$ is an alkyl group of $C_1$-$C_{18}$.

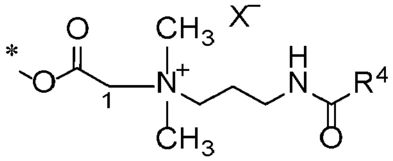

(Structure IV)

The positively charged organic group can derivatize the polysaccharide backbone at the 1, 2, 3, 4 and/or 6 positions of a glucose monomer in the backbone of the polysaccharide. Depending on reaction conditions and the specific substituent used to derivatize the polysaccharide, it is possible that the hydroxyl groups at certain glucose carbon positions may be disproportionately substituted due to steric hinderance and reactivity. For example, the hydroxyl at carbon position 6 for a branched unit may be more substituted than the hydroxyls at other carbon positions.

Depending on the reaction conditions and the specific substituent used to derivatize the polysaccharide backbone, certain glucose monomers on the backbone may be disproportionately substituted relative to other glucose monomers. Depending on reaction conditions and the specific substituent used, substitution of the polysaccharide may occur in a block manner.

The polysaccharide-based cationic polymers may comprise more than one type of positively charged organic group. In additional to the positively charged organic group, the polysaccharide-based cationic polymer may further comprise other function groups.

The average degree of substation (DoS) of the positive charged group is preferably from about 0.01 (one cationic charge per 100 polymer repeating units) to about 3 (three cationic charges per polymer repeating units), preferably from 0.03 to 2.0, more preferably from 0.05 to 1.0, and most preferably from 0.07 to 0.5. The term "degree of substitution" (DoS) as used herein refers to the average number of hydroxyl groups substituted in each monomeric unit (glucose) of a cationic polysaccharide.

One preferred family of polysaccharide-based cationic polymer is cationic modified cellulose. Suitable cationic modified celluloses include quaternized hydroxyethyl cellulose (Polyquaternium-10), which available under the tradename of Ucare by Dow Chemical, such as Ucare LR400, Ucare LR30M, Ucare JR125, Ucare JR400, etc. Suitable cationic modified cellulose polymers also include quaternised hydroxyethyl cellulose (HEC) polymers with cationic substitution of trimethyl ammonium and dimethyldodecyl ammonium (Polyquaternium-67), which available under trade the tradename of SoftCAT by Dow Chemical, such as SoftCAT SK, SoftCAT SK-MH, SoftCAT SX, SoftCAT SL. Other suitable cationic modified celluloses include those sold under tradename SupraCare™ by Dow Chemical, such as SupraCare™ 150, SupraCare™ 133, SupraCare™ 212, and polymer PK. Other suitable cationic modified cellulose polymers also include those modified with cationic group and/or a hydrophobic group and described as soil release polymers in WO2019111948, WO2019111949, WO2019111946 and WO2019111947. Suitable polymers may comprises more than one type of cationic modification, such as polymers disclosed in WO2022060754, WO2021242942 and WO2020/091988.

Suitable polysaccharide-based cationic polymer includes cationic modified dextran. Preferably, the cationic modified dextran is a dextran polymer functionalized with quaternary ammonium moieties; wherein the dextran polymer is a branched chain dextran polymer. More preferably, the cationic modified dextran is functionalized with quaternary ammonium moieties; wherein the dextran polymer comprises a branched chain dextran polymer; wherein the branched

chain dextran polymer comprises a plurality of glucose structural units; wherein 90 to 98 mol% (preferably, 92.5 to 97.5 mol%; more preferably, 93 to 97 mol%; most preferably, 94 to 96 mol%) of the glucose structural units are connected by a-D-1,6 linkages and 2 to 10 mol% (preferably, 2.5 to 7.5 mol%; more preferably, 3 to 7 mol%, most preferably, 4 to 6 mol%) of the glucose structural units are connected by a-1,3 linkages. Most preferably, the cationic modified dextran is functionalized with quaternary ammonium moieties; wherein the dextran polymer is a branched chain dextran polymer; wherein the branched chain dextran polymer comprises a plurality of glucose structural units; wherein 90 to 98 mol% (preferably, 92.5 to 97.5 mol%; more preferably, 93 to 97 mol%; most preferably, 94 to 96 mol%) of the glucose structural units are connected by a-D-1,6 linkages and 2 to 10 mol% (preferably, 2.5 to 7.5 mol%; more preferably, 3 to 7 mol%; most preferably, 4 to 6 mol%) of the glucose structural units are connected by a-1,3 linkages. Suitable cationic modified dextran polymers are described in WO2021194808. Suitable cationic modified dextran polymers are also commercially available under brand name CDC, CDC-L, CDC-H from Meito Sangyo, which has CAS number of 83855-79-2.

Suitable polysaccharide-based cationic polymer also includes cationic modified polyglucans. Suitable cationic modified polyglucans are based on alpha 1,3-polyglucans and/or 1,6-polyglucans.

In one embodiment, the cationic modified polyglucan is a poly alpha-1,3-glucan ether compound comprising:

(i) poly alpha-1,3-glucan substituted with at least one positively charged organic group;
(ii) a weight average degree of polymerization of at least 6; and
(iii) a degree of substitution of about 0.05 to about 3.0;

wherein the poly alpha-1,3-glucan comprises a backbone of glucose monomer units, and wherein at least 50% of the glucose monomer units are linked via alpha-1,3-glycosidic linkages.

Suitable cationic modified poly alpha-1,6-glucan ether is also described in WO2015095358 and WO2021225837.

In another embodiment, the cationic modified polyglucan is a poly alpha-1,6-glucan ether compound comprising:

(i) poly alpha-1 , 6-glucan substituted with at least one positively charged (ii) organic group;
(iii) a weight average degree of polymerization of at least 5; and a degree of substitution of about 0.001 to about 3.0; wherein the poly alpha-1 ,6-glucan comprises a backbone of glucose monomer units, and wherein at least 40% of the glucose monomer units are linked via alpha-1 ,6-glycosidic linkages.

Suitable cationic modified poly alpha-1,6-glucan ether is also described in WO2021/257786J, WO2021257793, WO2021257932.

In another embodiment, the cationic modified polyglucan is an ester derivative of a polyglucan, wherein the polyglucan has a degree of substitution (DoS) up to about 3.0 with at least one cationic organic group that is ester-linked to the polyglucan. Preferably, at least about 50% of the glycosidic linkages of the alpha-glucan are alpha-1,3-glycosidic linkages or alpha-1,6-glycosidic linkages. Suitable cationic modified poly glucan ester is described in WO2023/287684.

Another preferred family of polysaccharide-based cationic polymer is cationic modified guar. Suitable cationic modified guar include guar hydroxypropyltrimonium chloride, which available from by Ashland as AquaCat™ CG518 cationic solution, AquaCat™ PF618 cationic solution, N-Hance™ 3000, 3196, 3215, BF-13, BF-17, C261, C261N, CG13, CCG45. Other cationic modified guar polymers are available from Solvay as Jaguar® C 162, Excel, Excel SGI, Optima, C 13 S, C 13 SH, C14 S, C-17, LS SGI, C-500 STD. Other nonionic and/or anionic modified guar include for example Jaguar® HP 105 (Hydroxypropyl Guar gum), Jaguar® SOFT and HP-120 COS (Carboxymethyl Hydroxypropyl Guar Gum).

Another preferred family of polysaccharide-based cationic polymer is cationic modified inulin. "inulins" are understood to comprise polysaccharides comprising β-(2,1) linked fructofuranose units and a glucopyranose unit. The degree of polymerization ranges preferably between 2 and 60. Inulin can for example be obtained from chicory, dahlias and Jerusalem artichokes. Example of cationic modified inulin are as described in US20190274943, US20180119055. Suitable cationic modified inulin are Quatin series sold by Cosun Beet Company, including Quatin 350, Quatin 380 and Quatin 1280 which are characterized by different degree of substitution (DS), cationic density (meq/g) and molecular weight (g/mol).

Suitable polysaccharide-based cationic polymer includes cationic modified starch.

Fabric and home care compositions, such as laundry detergent compositions of the invention may contain further ingredients well-known to the person skilled in the art and can be prepared according to methods also well-known to the person skilled in the art.

Fabric and Home Care Composition: Any fabric and home care composition are suitable. Preferred are detergents and cleaning compositions. Especially preferred are fabric treatment compositions, even more preferred are laundry detergent compositions.

Fabric and home care compositions are typically suitable for: (a) the care of finished textiles, cleaning of finished textiles, sanitization of finished textiles, disinfection of finished textiles, detergents, stain removers, softeners, fabric

enhancers, stain removal or finished textiles treatments, pre and post wash treatments, washing machine cleaning and maintenance, with finished textiles intended to include garments and items made of cloth; (b) the care of dishes, glasses, crockery, cooking pots, pans, utensils, cutlery and the like in automatic, in-machine washing, including detergents, preparatory post treatment and machine cleaning and maintenance products for both the dishwasher, the utilized water and its contents; or (c) manual hand dish washing detergents.

The composition may j anionic soil release polymer and from 20wt% to 90wt% solvent, wherein the solvent is selected from the group consisting of water, ethanol, propanol, butanol, ethylene glycol, 1,2-propylene glycol, 1,3-propylene glycol, 1,2-butylene glycol, 1,3 butylene glycol, 1,4-butylene glycol, butyl glycol, butyl diglycol, butyl polyglycol, and any combination thereof.

The composition may be used to reduce the adhesion of soil to a fabric surface.

Laundry Detergent Composition: Suitable laundry detergent compositions include laundry detergent powder compositions, laundry beads, laundry detergent liquid compositions, laundry detergent gel compositions, laundry sheets, and water-soluble unit dose laundry detergent compositions.

Fabric Enhancers: Suitable fabric enhancers are liquid fabric enhancers including compact liquid fabric enhancers, and solid fabric enhancers including fabric enhancer beads.

Dish-washing Detergent Compositions: Suitable dish-washing detergent compositions include hand dish-washing detergent compositions and automatic dish-washing detergent compositions. Such as automatic dish-washing powder, tablet and pouches.

Hard Surface Cleaner Compositions: Suitable hard surface cleaner compositions include product that can be directly applied onto hard surface, eg. by a spray, and products that can be diluted in water before been applied onto hard surface.

Fabric and Home Care Ingredients: Suitable fabric and home care ingredients are described in more detail below.

Surfactant System: The compositions comprise a surfactant system in an amount sufficient to provide desired cleaning properties. In some embodiments, the composition comprises, by weight of the composition, from about 1% to about 70% of a surfactant system. In other embodiments, the composition comprises, by weight of the composition, from about 2% to about 60% of the surfactant system. In further embodiments, the composition comprises, by weight of the composition, from about 5% to about 30% of the surfactant system. The surfactant system may comprise a detersive surfactant selected from anionic surfactants, nonionic surfactants, cationic surfactants, zwitterionic surfactants, amphoteric surfactants, ampholytic surfactants, and mixtures thereof. Those of ordinary skill in the art will understand that a detersive surfactant encompasses any surfactant or mixture of surfactants that provide cleaning, stain removing, or laundering benefit to soiled material.

Suitable surfactants include anionic surfactants, non-ionic surfactant, cationic surfactants, zwitterionic surfactants and amphoteric surfactants and mixtures thereof. Suitable surfactants may be linear or branched, substituted or unsubstituted, and may be derived from petrochemical material or biomaterial. Preferred surfactant systems comprise both anionic and nonionic surfactant, preferably in weight ratios from 90:1 to 1:90. In some instances a weight ratio of anionic to nonionic surfactant of at least 1:1 is preferred. However, a ratio below 10:1 may be preferred. When present, the total surfactant level is preferably from 0.1% to 60%, from 1% to 50% or even from 5% to 40% by weight of the subject composition.

Anionic Surfactant: Anionic surfactants include, but are not limited to, those surface-active compounds that contain an organic hydrophobic group containing generally 8 to 22 carbon atoms or generally 8 to 18 carbon atoms in their molecular structure and at least one water-solubilizing group preferably selected from sulfonate, sulfate, and carboxylate so as to form a water-soluble compound. Usually, the hydrophobic group will comprise a $C_8$-$C_{22}$ alkyl, or acyl group. Such surfactants are employed in the form of water-soluble salts and the salt-forming cation usually is selected from sodium, potassium, ammonium, magnesium and mono-, with the sodium cation being the usual one chosen.

Anionic surfactants of the present invention and adjunct anionic cosurfactants, may exist in an acid form, and said acid form may be neutralized to form a surfactant salt which is desirable for use in the present detergent compositions. Typical agents for neutralization include the metal counterion base such as hydroxides, e.g., NaOH or KOH. Further preferred agents for neutralizing anionic surfactants of the present invention and adjunct anionic surfactants or cosurfactants in their acid forms include ammonia, amines, oligamines, or alkanolamines. Alkanolamines are preferred. Suitable non-limiting examples including monoethanolamine, diethanolamine, triethanolamine, and other linear or branched alkanolamines known in the art; for example, highly preferred alkanolamines include 2-amino-1-propanol, 1-aminopropanol, monoisopropanolamine, or 1-amino-3-propanol. Amine neutralization may be done to a full or partial extent, e.g. part of the anionic surfactant mix may be neutralized with sodium or potassium and part of the anionic surfactant mix may be neutralized with amines or alkanolamines.

Suitable sulphonate surfactants include methyl ester sulphonates, alpha olefin sulphonates, alkyl benzene sulphonates, especially alkyl benzene sulphonates, preferably $C_{10}$-$C_{13}$ alkyl benzene sulphonate. Suitable alkyl benzene sulphonate (LAS) is obtainable, preferably obtained, by sulphonating commercially available linear alkyl benzene (LAB). Suitable LAB includes low 2-phenyl LAB, such as those supplied by Sasol under the tradename Isochem® or those

supplied by Petresa under the tradename Petrelab®, other suitable LAB include high 2-phenyl LAB, such as those supplied by Sasol under the tradename Hyblene®. A suitable anionic surfactant is alkyl benzene sulphonate that is obtained by DETAL catalyzed process, although other synthesis routes, such as HF, may also be suitable. In one aspect a magnesium salt of LAS is used.

Preferably, the composition may contain from about 0.5% to about 30%, by weight of the laundry composition, of an HLAS surfactant selected from alkyl benzene sulfonic acids, alkali metal or amine salts of $C_{10}$-$C_{16}$ alkyl benzene sulfonic acids, wherein the HLAS surfactant comprises greater than 50% $C_{12}$, preferably greater than 60%, preferably greater than 70% $C_{12}$, more preferably greater than 75%

Suitable sulphate surfactants include alkyl sulphate, preferably $C_{8-18}$ alkyl sulphate, or predominantly $C_{12}$ alkyl sulphate.

A preferred sulphate surfactant is alkyl alkoxylated sulphate, preferably alkyl ethoxylated sulphate, preferably a $C_8$-$C_{18}$ alkyl alkoxylated sulphate, preferably a $C_8$-$C_{18}$ alkyl ethoxylated sulphate, preferably the alkyl alkoxylated sulphate has an average degree of alkoxylation of from 0.5 to 20, preferably from 0.5 to 10, preferably the alkyl alkoxylated sulphate is a $C_8$-$C_{18}$ alkyl ethoxylated sulphate having an average degree of ethoxylation of from 0.5 to 10, preferably from 0.5 to 5, more preferably from 0.5 to 3 or from about 1.5 to 3 or from about 1.8 to 2.5. The alkyl alkoxylated sulfate may have a broad alkoxy distribution or a peaked alkoxy distribution. The alkyl portion of the AES may include, on average, from 13.7 to about 16 or from 13.9 to 14.6 carbons atoms. At least about 50% or at least about 60% of the AES molecule may include having an alkyl portion having 14 or more carbon atoms, preferable from 14 to 18, or from 14 to 17, or from 14 to 16, or from 14 to 15 carbon atoms.

The alkyl sulphate, alkyl alkoxylated sulphate and alkyl benzene sulphonates may be linear or branched, including 2 alkyl substituted or mid chain branched type, substituted or un-substituted, and may be derived from petrochemical material or biomaterial. Preferably, the branching group is an alkyl. Typically, the alkyl is selected from methyl, ethyl, propyl, butyl, pentyl, cyclic alkyl groups and mixtures thereof. Single or multiple alkyl branches could be present on the main hydrocarbyl chain of the starting alcohol(s) used to produce the sulfated anionic surfactant used in the detergent of the invention. Most preferably the branched sulfated anionic surfactant is selected from alkyl sulfates, alkyl ethoxy sulfates, and mixtures thereof.

Alkyl sulfates and alkyl alkoxy sulfates are commercially available with a variety of chain lengths, ethoxylation and branching degrees. Commercially available sulfates include those based on Neodol alcohols ex the Shell company, Lial - Isalchem and Safol ex the Sasol company, natural alcohols ex The Procter & Gamble Chemicals company.

Other suitable anionic surfactants include alkyl ether carboxylates, comprising a $C_{10}$-$C_{26}$ linear or branched, preferably $C_{10}$-$C_{20}$ linear, most preferably $C_{16}$-$C_{18}$ linear alkyl alcohol and from 2 to 20, preferably 7 to 13, more preferably 8 to 12, most preferably 9.5 to 10.5 ethoxylates. The acid form or salt form, such as sodium or ammonium salt, may be used, and the alkyl chain may contain one cis or trans double bond. Alkyl ether carboxylic acids are available from Kao (Akypo®), Huntsman (Empicol®) and Clariant (Emulsogen®).

Other suitable anionic surfactants are rhamnolipids. The rhamnolipids may have a single rhamnose sugar ring or two rhamnose sugar rings.

Non-ionic Surfactant: Suitable non-ionic surfactants are selected from the group consisting of: $C_8$-$C_{18}$ alkyl ethoxylates, such as, NEODOL® non-ionic surfactants from Shell; $C_6$-$C_{12}$ alkyl phenol alkoxylates wherein preferably the alkoxylate units are ethyleneoxy units, propyleneoxy units or a mixture thereof; $C_{12}$-$C_{18}$ alcohol and $C_6$-$C_{12}$ alkyl phenol condensates with ethylene oxide/propylene oxide block polymers such as Pluronic® from BASF; alkylpolysaccharides, preferably alkylpolyglycosides; methyl ester ethoxylates; polyhydroxy fatty acid amides; ether capped poly(oxyalkylated) alcohol surfactants; and mixtures thereof.

Suitable non-ionic surfactants are alkylpolyglucoside and/or an alkyl alkoxylated alcohol.

Suitable non-ionic surfactants include alkyl alkoxylated alcohols, preferably $C_8$-$C_{18}$ alkyl alkoxylated alcohol, preferably a $C_8$-$C_{18}$ alkyl ethoxylated alcohol, preferably the alkyl alkoxylated alcohol has an average degree of alkoxylation of from 1 to 50, preferably from 1 to 30, or from 1 to 20, or from 1 to 10, preferably the alkyl alkoxylated alcohol is a $C_8$-$C_{18}$ alkyl ethoxylated alcohol having an average degree of ethoxylation of from 1 to 10, preferably from 1 to 7, more preferably from 1 to 5 and most preferably from 3 to 7. In one aspect, the alkyl alkoxylated alcohol is a $C_{12}$-$C_{15}$ alkyl ethoxylated alcohol having an average degree of ethoxylation of from 7 to 10. The alkyl alkoxylated alcohol can be linear or branched, and substituted or un-substituted. Suitable nonionic surfactants include those with the trade name Lutensol® from BASF. The alkyl alkoxylated sulfate may have a broad alkoxy distribution for example Alfonic 1214-9 Ethoxylate or a peaked alkoxy distribution for example Novel 1214-9 both commercially available from Sasol.

Cationic surfactant: Suitable cationic surfactants include alkyl pyridinium compounds, alkyl quaternary ammonium compounds, alkyl quaternary phosphonium compounds, alkyl ternary sulphonium compounds, and mixtures thereof.

Preferred cationic surfactants are quaternary ammonium compounds having the general formula:

$$(R)(R_1)(R_2)(R_3)N^+X^-$$

wherein, R is a linear or branched, substituted or unsubstituted $C_{6-18}$ alkyl or alkenyl moiety, $R_1$ and $R_2$ are independently selected from methyl or ethyl moieties, $R_3$ is a hydroxyl, hydroxymethyl or a hydroxyethyl moiety, X is an anion which provides charge neutrality, preferred anions include: halides, preferably chloride; sulphate; and sulphonate.

The fabric care compositions of the present invention may contain up to about 30%, alternatively from about 0.01% to about 20%, more alternatively from about 0.1% to about 20%, by weight of the composition, of a cationic surfactant. For the purposes of the present invention, cationic surfactants include those which can deliver fabric care benefits. Non-limiting examples of useful cationic surfactants include: fatty amines, imidazoline quat materials and quaternary ammonium surfactants, preferably N, N-bis(stearoyl-oxy-ethyl) N,N-dimethyl ammonium chloride, N,N-bis(tallowoyl-oxy-ethyl) N,N-dimethyl ammonium chloride, N,N-bis(stearoyl-oxy-ethyl) N-(2 hydroxyethyl) N-methyl ammonium methylsulfate; 1, 2 di (stearoyl-oxy) 3 trimethyl ammoniumpropane chloride; dialkylenedimethylammonium salts such as dicanoladimethylammonium chloride, di(hard)tallowdimethylammonium chloride dicanoladimethylammonium methyl sulfate; 1-methyl-1-stearoylamidoethyl-2-stearoylimidazolinium methylsulfate; 1-tallowylamidoethyl-2-tallowylimidazoline; N,N"-dialkyldiethylenetriamine ;the reaction product of N-(2-hydroxyethyl)-1,2-ethylenediamine or N-(2-hydroxyisopropyl)-1,2-ethylenediamine with glycolic acid, esterified with fatty acid, where the fatty acid is (hydrogenated) tallow fatty acid, palm fatty acid, hydrogenated palm fatty acid, oleic acid, rapeseed fatty acid, hydrogenated rapeseed fatty acid; polyglycerol esters (PGEs), oily sugar derivatives, and wax emulsions and a mixture of the above.

It will be understood that combinations of softener actives disclosed above are suitable for use herein.

Amphoteric and Zwitterionic surfactant: Suitable amphoteric or zwitterionic surfactants include amine oxides, and/or betaines. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one $R^1$ $C_8$-$C_{18}$ alkyl moiety and 2 $R^2$ and $R^3$ moieties selected from the group consisting of $C_1$-$C_3$ alkyl groups and $C_1$-$C_3$ hydroxyalkyl groups. Preferably amine oxide is characterized by the formula $R^1$-$N(R^2)(R^3)O$ wherein $R^1$ is a $C_8$-$C_{18}$ alkyl and $R^2$ and $R^3$ are selected from the group consisting of methyl, ethyl, propyl, isopropyl, 2-hydroxethyl, 2-hydroxypropyl and 3-hydroxypropyl. The linear amine oxide surfactants in particular may include linear $C_{10}$-$C_{18}$ alkyl dimethyl amine oxides and linear $C_8$-$C_{12}$ alkoxy ethyl dihydroxy ethyl amine oxides.

Other suitable surfactants include betaines, such as alkyl betaines, alkylamidobetaine, amidazoliniumbetaine, sulfobetaine (INCI Sultaines) as well as Phosphobetaines.

Other Cleaning Additives: The compositions of the invention may also contain other cleaning additives. Suitable cleaning additives include enzymes, builders, structurants or thickeners, polymers, additional amines, bleaching agents, fluorescent brightener, fabric hueing agents, chelating agent, encapsulates, perfume, malodor reduction materials, conditioning agents, probiotics, organic acids, anti-oxidant, hygiene Agent, pearlescent agent, opacifier, solvents, hydrotrope, suds suppressor.

Enzymes: Preferably the composition comprises one or more enzymes. Preferred enzymes provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, mannanases, galactanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is an enzyme cocktail that may comprise, for example, a protease and lipase in conjunction with amylase. When present in the composition, the aforementioned additional enzymes may be present at levels from about 0.00001% to about 2%, from about 0.0001% to about 1% or even from about 0.001% to about 0.5% enzyme protein by weight of the composition.

Proteases. Preferably the composition comprises one or more proteases. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

(a) subtilisins (EC 3.4.21.62), especially those derived from *Bacillus,* such as *Bacillus sp.,* Bacillus sp., B. lentus, B. alkalophilus, B. subtilis, B. amyloliquefaciens, B. gibsonii, B. akibaii, B. clausii and B. clarkii described in WO2004067737, WO2015091989, WO2015091990, WO2015024739, WO2015143360, US6,312,936B1, US5,679,630, US4,760,025, DE102006022216A1, DE102006022224A1, WO2015089447, WO2015089441, WO2016066756, WO2016066757, WO2016069557, WO2016069563, WO2016069569, WO2017/089093, WO2020/156419.

(b) trypsin-type or chymotrypsin-type proteases, such as trypsin (e.g., of porcine or bovine origin), including the *Fusarium* protease described in WO 89/06270 and the chymotrypsin proteases derived from *Cellumonas* described in

WO 05/052161 and WO 05/052146.

(c) metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces spp.* Described in WO2014194032, WO2014194054 and WO2014194117; from *Kribella alluminosa* described in WO2015193488; and from *Streptomyces* and *Lysobacter* described in WO2016075078.

(d) Protease having at least 90% identity to the subtilase from *Bacillus sp.* TY145, NCIMB 40339, described in WO92/17577 (Novozymes A/S), including the variants of this *Bacillus sp* TY145 subtilase described in WO2015024739, and WO2016066757.

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Liquanase® Evity®, Savinase® Evity®, Ovozyme®, Neutrase®, Everlase®, Coronase®, Blaze®, Blaze Ultra®, Blazed Evity®, Blazed Exceed, Blazed Pro, Esperase®, Progress® Uno, Progress® Excel, Progress® Key, Ronozyme®, Vinzon® and Het Ultra® by Novozymes A/S (Denmark); those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont; those sold under the tradename Opticlean® and Optimase® by Solvay Enzymes; and those available from Henkel/Kemira, namely BLAP (sequence shown in Figure29 of US 5,352,604 with the following mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and KAP (*Bacillus alkalophilus subtilisin* with mutations A230V + S256G + S259N) from Kao and Lavergy®, Lavergy® Pro, Lavergy® C Bright from BASF.

Amylases. Preferably the composition may comprise an amylase. Suitable alpha-amylases include those of bacterial or fungal origin. Chemically or genetically modified mutants (variants) are included. A preferred alkaline alpha-amylase is derived from a strain of *Bacillus,* such as *Bacillus licheniformis, Bacillus amyloliquefaciens, Bacillus stearothermophilus, Bacillus subtilis,* or other *Bacillus sp.,* such as *Bacillus sp.* NCIB 12289, NCIB 12512, NCIB 12513, DSM 9375 (USP 7,153,818) DSM 12368, DSMZ no. 12649, KSM AP1378 (WO 97/00324), KSM K36 or KSM K38 (EP 1,022,334). Preferred amylases include:

(a) variants described in WO 94/02597, WO 94/18314, WO96/23874 and WO 97/43424, especially the variants with substitutions in one or more of the following positions versus the enzyme listed as SEQ ID No. 2 in WO 96/23874: 15, 23, 105, 106, 124, 128, 133, 154, 156, 181 , 188, 190, 197, 202, 208, 209, 243, 264, 304, 305, 391, 408, and 444.

(b) variants described in USP 5,856,164 and WO99/23211, WO 96/23873, WO00/60060 and WO 06/002643, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 203, 214, 231, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.

(c) variants exhibiting at least 90% identity with SEQ ID No. 4 in WO06/002643, the wild-type enzyme from *Bacillus SP722,* especially variants with deletions in the 183 and 184 positions and variants described in WO 00/60060, which is incorporated herein by reference.

(d) variants exhibiting at least 95% identity with the wild-type enzyme from *Bacillus sp.707* (SEQ ID NO:7 in US 6,093, 562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations.

(e) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.

(f) variants exhibiting at least 89% identity with SEQ ID NO:1 in WO2016091688, especially those comprising deletions at positions H183+G184 and additionally one or more mutations at positions 405, 421, 422 and/or 428.

(g) variants exhibiting at least 60% amino acid sequence identity with the "PcuAmyl $\alpha$-amylase" from *Paenibacillus curdlanolyticus* YK9 (SEQ ID NO:3 in WO2014099523).

(h) variants exhibiting at least 60% amino acid sequence identity with the "CspAmy2 amylase" from *Cytophaga sp.* (SEQ ID NO:1 in WO2014164777).

(i) variants exhibiting at least 85% identity with AmyE from Bacillus subtilis (SEQ ID NO:1 in WO2009149271).

(j) Variants exhibiting at least 90% identity variant with the wild-type amylase from Bacillus sp. KSM-K38 with accession number AB051102.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL® and BAN® (Novozymes A/S, Bagsvaerd, Denmark), KEMZYM® AT 9000 Biozym Biotech Trading GmbH Wehlistrasse 27b A-1200 Wien Austria,

RAPIDASE® , PURASTAR®, ENZYSIZE®, OPTISIZE HT PLUS®, POWERASE® and PURASTAR OXAM® (Genencor International Inc., Palo Alto, California) and KAM® (Kao, 14-10 Nihonbashi Kayabacho, 1-chome, Chuo-ku Tokyo 103-8210, Japan). In one aspect, suitable amylases include NATALASE®, STAINZYME® and STAINZYME PLUS® and mixtures thereof.

Lipases. Preferably the composition comprises one or more lipases, including "first cycle lipases" such as those described in U.S. Patent 6,939,702 B1 and US PA 2009/0217464. Preferred lipases are first-wash lipases. In one embodiment of the invention the composition comprises a first wash lipase.

First wash lipases includes a lipase which is a polypeptide having an amino acid sequence which: (a) has at least 90% identity with the wild-type lipase derived from *Humicola lanuginosa* strain DSM 4109; (b) compared to said wild-type lipase, comprises a substitution of an electrically neutral or negatively charged amino acid at the surface of the three-dimensional structure within 15A of E1 or Q249 with a positively charged amino acid; and (c) comprises a peptide addition at the C-terminal; and/or (d) comprises a peptide addition at the N-terminal and/or (e) meets the following limitations: i) comprises a negative amino acid in position E210 of said wild-type lipase; ii) comprises a negatively charged amino acid in the region corresponding to positions 90-101 of said wild-type lipase; and iii) comprises a neutral or negative amino acid at a position corresponding to N94 or said wild-type lipase and/or has a negative or neutral net electric charge in the region corresponding to positions 90-101 of said wild-type lipase.

Preferred are variants of the wild-type lipase from Thermomyces lanuginosus comprising one or more of the T231R and N233R mutations. The wild-type sequence is the 269 amino acids (amino acids 23 - 291) of the Swissprot accession number Swiss-Prot O59952 (derived from Thermomyces lanuginosus (Humicola lanuginosa)). Other suitable lipases include: Liprl 139, e.g. as described in WO2013/171241; TfuLip2, e.g. as described in WO2011/084412 and WO2013/033318; Pseudomonas stutzeri lipase, e.g. as described in WO2018228880; Microbulbifer thermotolerans lipase, e.g. as described in WO2018228881; Sulfobacillus acidocaldarius lipase, e.g. as described in EP3299457; LIP062 lipase e.g. as described in WO2018209026; PinLip lipase e.g. as described in WO2017036901 and Absidia sp. lipase e.g. as described in WO2017005798.

Preferred lipases would include those sold under the tradenames Lipex® and Lipolex® and Lipoclean®.

Cellulases. Suitable enzymes include cellulases of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263 , US 5,691,178 , US 5,776,757 and US 5,691,178 . Suitable cellulases include the alkaline or neutral cellulases having colour care benefits. Commercially available cellulases include CELLUZYME®, CAREZYME® and CAREZYME PREMIUM (Novozymes A/S), CLAZINASE®, and PURADAX HA® (Genencor International Inc.), and KAC-500(B)® (Kao Corporation).

The bacterial cleaning cellulase may be a glycosyl hydrolase having enzymatic activity towards amorphous cellulose substrates, wherein the glycosyl hydrolase is selected from GH families 5, 7, 12, 16, 44 or 74. Suitable glycosyl hydrolases may also be selected from the group consisting of: GH family 44 glycosyl hydrolases from *Paenibacillus polyxyma* (wild-type) such as XYG1006 described in US 7,361,736 or are variants thereof. GH family 12 glycosyl hydrolases from *Bacillus licheniformis* (wild-type) such as SEQ ID NO:1 described in US 6,268,197 or are variants thereof; GH family 5 glycosyl hydrolases from *Bacillus agaradhaerens* (wild type) or variants thereof; GH family 5 glycosyl hydrolases from *Paenibacillus* (wild type) such as XYG1034 and XYG 1022 described in US 6,630,340 or variants thereof; GH family 74 glycosyl hydrolases from *Jonesia sp.* (wild type) such as XYG1020 described in WO 2002/077242 or variants thereof; and GH family 74 glycosyl hydrolases from *Trichoderma Reesei* (wild type), such as the enzyme described in more detail in Sequence ID NO. 2 of US 7,172,891 , or variants thereof. Suitable bacterial cleaning cellulases are sold under the tradenames Celluclean® and Whitezyme® (Novozymes A/S, Bagsvaerd, Denmark).

The composition may comprise a fungal cleaning cellulase belonging to glycosyl hydrolase family 45 having a molecular weight of from 17kDa to 30 kDa, for example the endoglucanases sold under the tradename Biotouch® NCD, DCC and DCL (AB Enzymes, Darmstadt, Germany).

Pectate Lyases. Other preferred enzymes include pectate lyases sold under the tradenames Pectawash®, Pectaway®, Xpect® and mannanases sold under the tradenames Mannaway® (all from Novozymes A/S, Bagsvaerd, Denmark), and Purabrite® (Genencor International Inc., Palo Alto, California).

Nucleases. The composition may comprise a nuclease enzyme. The nuclease enzyme is an enzyme capable of cleaving the phosphodiester bonds between the nucleotide sub-units of nucleic acids. The nuclease enzyme herein is preferably a deoxyribonuclease or ribonuclease enzyme or a functional fragment thereof. By functional fragment or part is meant the portion of the nuclease enzyme that catalyzes the cleavage of phosphodiester linkages in the DNA backbone and so is a region of said nuclease protein that retains catalytic activity. Thus, it includes truncated, but functional versions, of the enzyme and/or variants and/or derivatives and/or homologues whose functionality is maintained. Suitable DNases include wild-types and variants described in detail by WO2017162836 and WO2018108865, and variants of the Bacillus cibi DNase including those described in WO2018011277.

RNase: suitable RNases include wild-types and variants of DNases described in WO2018178061 and

WO2020074499.

Preferably the nuclease enzyme is a deoxyribonuclease, preferably selected from any of the classes E.C. 3.1.21.x, where x=1, 2, 3, 4, 5, 6, 7, 8 or 9, E.C. 3.1.22.y where y=1, 2, 4 or 5, E.C. 3.1.30.z where z= 1 or 2, E.C. 3.1.31.1 and mixtures thereof.

Hexosaminidases. The composition may comprise one or more hexosaminidases. The term hexosaminidase includes "dispersin" and the abbreviation "Dsp", which means a polypeptide having hexosaminidase activity, EC 3.2.1 .- that catalyzes the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 may be preferred, especially the variants with improved thermostability disclosed in that publication.

Mannanases. The composition may comprise an extracellular-polymer-degrading enzyme that includes a mannanase enzyme. The term "mannanase" means a polypeptide having mannan endo-1,4-beta-mannosidase activity (EC 3.2.1.78) from the glycoside hydrolase family 26 that catalyzes the hydrolysis of 1,4-3-D-mannosidic linkages in mannans, galactomannans and glucomannans. Alternative names of mannan endo-1,4-beta-mannosidase are 1,4-3-D-mannan mannanohydrolase; endo-1,4-3-mannanase; endo- β-1,4-mannase; β-mannanase B; 3-1,4-mannan 4-mannanohydrolase; endo-3-mannanase; and β-D-mannanase. For purposes of the present disclosure, mannanase activity may be determined using the Reducing End Assay as described in the experimental section of WO2015040159. Suitable examples from class EC 3.2.1.78 are described in WO2015040159, such as the mature polypeptide SEQ ID NO: 1 described therein.

Galactanases. The composition may comprise an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

Enzyme Stabilizing System: The composition may optionally comprise from about 0.001% to about 10%, in some examples from about 0.005% to about 8%, and in other examples, from about 0.01% to about 6%, by weight of the composition, of an enzyme stabilizing system. The enzyme stabilizing system can be any stabilizing system which is compatible with the detersive enzyme. In the case of aqueous detergent compositions comprising protease, a reversible protease inhibitor, such as a boron compound, including borate, 4-formyl phenylboronic acid, phenylboronic acid and derivatives thereof, or compounds such as calcium formate, sodium formate and 1,2-propane diol may be added to further improve stability.

Builders: The composition may optionally comprise a builder. Built compositions typically comprise at least about 1% builder, based on the total weight of the composition. Liquid compositions may comprise up to about 10% builder, and in some examples up to about 8% builder, of the total weight of the composition. Granular compositions may comprise up to about 30% builder, and in some examples up to about 5% builder, by weight of the composition.

Builders selected from aluminosilicates (e.g., zeolite builders, such as zeolite A, zeolite P, and zeolite MAP) and silicates assist in controlling mineral hardness in wash water, especially calcium and/or magnesium, or to assist in the removal of particulate soils from surfaces. Suitable builders may be selected from the group consisting of phosphates, such as polyphosphates (e.g., sodium tri-polyphosphate), especially sodium salts thereof; carbonates, bicarbonates, sesquicarbonates, and carbonate minerals other than sodium carbonate or sesquicarbonate; organic mono-, di-, tri-, and tetracarboxylates, especially water-soluble nonsurfactant carboxylates in acid, sodium, potassium or alkanolammonium salt form, as well as oligomeric or water-soluble low molecular weight polymer carboxylates including aliphatic and aromatic types; and phytic acid. These may be complemented by borates, e.g., for pH-buffering purposes, or by sulfates, especially sodium sulfate and any other fillers or carriers which may be important to the engineering of stable surfactant and/or builder-containing compositions. Additional suitable builders may be selected from citric acid, lactic acid, fatty acid and salt thereof.

Suitable builders may include polycarboxylate and salt thereof, for example, homopolymers of acrylic acid, copolymers of acrylic acid and maleic acid, and copolymers of acrylic acid and/or maleic acid, and other suitable ethylenic monomers with various types of additional functionalities. More suitable polycarboxylate are described in polycarboxylate polymers section of this patent.

Also suitable for use as builders herein are synthesized crystalline ion exchange materials or hydrates thereof having chain structure and a composition represented by the following general anhydride form: $x(M_2O)\cdot ySiO_2\cdot zM'O$ wherein M is Na and/or K, M' is Ca and/or Mg; y/x is 0.5 to 2.0; and z/x is 0.005 to 1.0.

Alternatively, the composition may be substantially free of builder.

Structurant / Thickeners: Suitable structurant / thickeners include:

i. Di-benzylidene Polyol Acetal Derivative
ii. Bacterial Cellulose
iii. Coated Bacterial Cellulose
iv. Cellulose fibers non-bacterial cellulose derived
v. Non-Polymeric Crystalline Hydroxyl-Functional Materials
vi. Polymeric Structuring Agents
vii. Di-amido-gellants
viii. Any combination of above.

Polymers:

The compositions may include one or more additional polymers. Typically, the level of polymers is from about 0.01% to about 10.0 % by weight of the composition, preferably from about 0.1% to about 5%, and more preferably from about 0.2% to about 3.0% by weight of the composition. In some situations where the composition is in concentrated form, such as concentrated fabric and home care products in any forms which designed for consumer to dilute at home and then use following their regular dosing habits, the level of the polymers maybe higher than 10.0%, or higher than 5.0%, by weight of the composition.

Depending on the structure of the polymer, polymers can provide various benefits for the composition, including but not limit to, hydrophobic and hydrophilic stain removal, surfactant boosting, soil suspension, whiteness maintenance, soil release, malodor control, dye transfer inhibition, enhanced softness, enhanced freshness, etc. Polymers are normally multi-functional, which means one specific given type of polymer may provide more than one types of benefit as mentioned above. For example, a specific soil release polymer may provide soil release benefit as primary benefit, while also providing other benefits such as whiteness maintenance, malodor control, soil suspension, dye transfer inhibition.

Suitable polymers including, but not limited to the following:

Graft Polymers Based on Polyalkylene Oxide. The composition may comprise graft polymers which comprising polyalkylene oxide backbone (A) as a graft base and polymeric sidechains (B) grafted thereon. The polymeric sidechains (B) are obtainable by polymerization of at least one vinyl ester monomer. The polyalkylene oxide backbone (A) is obtainable by polymerization of at least one monomers selected from the group of ethylene oxide, 1 ,2-propylene oxide, 1 ,2-butylene oxide, 2,3-butylene oxide, 1 ,2-pentene oxide or 2,3-pentene oxide. Such graft polymers are known as effective soil suspension polymers for hydrophobic and hydrophilic stains, surfactant boosters, and sometimes as dye transfer inhibitors.

Suitable graft polymers include amphilic graft co-polymer comprises polyethylene glycol backbone (A) as a graft base, and at least one pendant sidechains (B) selected from polyvinyl acetate, polyvinyl alcohol and mixtures thereof. A preferred graft polymer of this type is Sokalan HP22 available from BASF.

Suitable graft polymers are also described in WO2007/138053 as amphiphilic graft polymers based on water-soluble polyalkylene oxides (A) as a graft base and side chains formed by polymerization of a vinyl ester component (B), said polymers having an average of < one graft site per 50 alkylene oxide units and mean molar masses M of from 3 000 to 100 000. One specific preferred graft polymer of this type is polyvinyl acetate grafted polyethylene oxide copolymer having a polyethylene oxide as graft base and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units. The most preferred polymer of this type is available from BASF as Sokalan PG101.

Suitable graft polymer also include graft polymer comprising a block copolymer backbone (A) as a graft base, wherein said block copolymer backbone (A) is obtainable by polymerization of at least two monomers selected from the group of ethylene oxide, 1 ,2-propylene oxide, 1 ,2-butylene oxide, 2,3-butylene oxide, 1 ,2-pentene oxide or 2,3-pentene oxide, wherein the number (x) of individual blocks within the block copolymer backbone (A) is an integer, wherein x is from 2 to 10 and preferably 3 to 5, and (B) polymeric sidechains grafted onto the block copolymer backbone, wherein said polymeric sidechains (B) are obtainable by polymerization of at least one vinyl ester monomer. Suitable graft polymers of this type are described in WO2021/160795 and WO2021/160851, these polymers have improved biodegradation profiles.

Suitable graft polymer also include graft polymer comprising a polyalkylene oxide backbone (A) which has a number average molecular weight of from about 1000 to about 20,000 Daltons and is based on ethylene oxide, propylene

oxide, or butylene oxide; and side chains derived from N-vinylpyrrolidone (B), and side chains derived from vinyl ester (C) derived from a saturated monocarboxylic acid containing from 1 to 6 carbon atoms and/or a methyl or ethyl ester of acrylic or methacrylic acid. Such graft polymers are described in WO2020005476 and can be used as dye transfer inhibitors.

Modified Polyamine Dispersing Agent

The composition may comprise one or more modified polyamine dispersing agent. The modified polyamine dispersant comprises a polyamine core structure and a plurality of alkoxylate groups attached to the core structure. The polyamine core structure includes polyalkyleneimine, and linear or branched oligoamine.

The polyamine core structure and the alkoxylate groups attached to the core structure can be further derivatized. For example, the polyamine core structure can be further partly or completely quaternized with $C_1$-$C_{30}$ linear or branched alkyl, more preferably $C_1$-$C_{10}$ or even $C_1$-$C_5$ linear or branched alkyl, most preferably methyl. The alkoxylate group can be further sulphated, sulphonated and/or substituted with an amino functional group.

Suitable modified polyamine dispersing agent includes ethoxylated polyethyleneimine (EPEI). EPEI are effective dispersing agent for hydrophilic stains, especially hydrophilic particulate stain such as clay.

In one embodiment, the EPEI has a polyethyleneimine backbone of weight average molecular weight of between 100g/mol and 2000g/mol, preferably between 200g/mol and 1500g/mol, more preferably between 300g/mol and 1000g/mol, even more preferably between 400g/mol and 800g/mol, most preferably between 500g/mol and 700g/mol, preferably about 600. The ethoxylation chains within the EPEI may be from 200g/mol to 2000g/mol weight average molecular weight, preferably from 400g/mol to 1500g/mol weight average molecular weight, more preferably from 600g/mol to 1000g/mol weight average molecular weight, most preferably about 880g/mol weight average molecular weight per ethoxylated chain. The ethoxylation chains within the EPEI have on average 5 to 40, preferably 10 to 30, more preferably 15 to 25, even more preferably 18 to 22, most preferably about 20 ethoxy units per ethoxylation chain. The EPEI may have a total weight average molecular weight of from 5000g/mol to 20000g/mol, preferably from 7500g/mol to 17500g/mol, more preferably from 10000g/mol to 15000g/mol, even more preferably from 12000g/mol to 13000g/mol, most preferably about 12700g/mol. A preferred example is polyethyleneimine core (with average molecular weight about 600g/mol) ethoxylated to 20 EO groups per NH. Suitable EPEI this type includes Sokalan HP20 available from BASF, Lutensol FP620 from BASF. Examples of available polyethyleneimine ethoxylates also include those prepared by reacting ethylene oxide with Epomine SP-006 manufactured by Nippon Shokubai.

In another embodiment, the EPEI comprises polyethyleneimine has an average molecular weight (Mw) ranging from 1800 to 5000 g/mol (prior to ethoxylation), and the polyoxyethylene side chains have an average of from 25 to 40 ethoxy units per side chain bonded to the polyethyleneimine backbone. Such EPEI is described in WO2020/030760 and WO2020/030469.

Suitable modified polyamine dispersing agent includes amphiphilic alkoxylated polyalkyleneimine polymer. These polymers have balanced hydrophilic and hydrophobic properties such that they remove grease and body soil particles from fabrics and surfaces, and keep the particles suspended in washing liquor. Suitable amphiphilic water-soluble alkoxylated polyalkyleneimine polymer is described in WO2009/061990 and WO2006/108857, which comprising in polyalkyleneimine, preferable polyethyleneimine core, and alkoxylate group of below connected to the core

$$*\text{-}[A^2\text{-}O]_m\text{-}[CH_2CH_2O]_n\text{-}[A^3\text{-}O]_p\text{-}R \qquad (V)$$

wherein

"*" in each case denotes one-half of bond to the nitrogen atom of the core.
$A^2$ is in each case independently selected from 1,2-propylene, 1,2-butylene, and 1,2-isobutylene;
$A^3$ is 1,2-propylene;
R is in each case independently selected from hydrogen and $C_1$-$C_4$-alkyl, preferably hydrogen;
m has an average value in the range of from 0 to 2, preferably 0;
n has an average value in the range of 5 to 50; and
p has an average value in the range of 3-50;

The polymer comprising a degree of quaterization ranging from 0 to 50, preferably from 0 to 20, and more preferably from 0 to 10.

A preferred alkoxylated polyalkyleneimine polymer is polyethyleneimine (MW = 600) modified with 24 ethoxylate groups per -NH and 16 propoxylate groups per -NH. Another preferred alkoxylated polyalkyleneimine polymer is polyethyleneimine (MW = 600) modified with 10 ethoxylate groups per -NH and 7 propoxylate groups per -NH.

Suitable alkoxylated polyalkyleneimine polymer of this type includes Sokalan HP30 Booster available from BASF.

Another Suitable modified polyamine dispersing agent is described in WO2021061774.

Suitable modified polyamine dispersing agent also includes zwitterionic polyamines. Said zwitterionic polyamine is selected from zwitterionic polyamines according to the following formula:

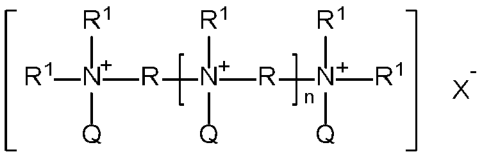

R is each independently $C_3$-$C_{20}$ linear or branched alkylene;

$R^1$ is an anionic unit-capped polyalkyleneoxy unit having the formula: $-(R^2O)_xR^3$,

wherein

$R^2$ is $C_2$-$C_4$ linear or branched alkylene, preferably $C_2$ (ethylene);

$R^3$ is hydrogen, an anionic unit, and mixtures thereof, in which not all $R^3$ groups are hydrogen, preferably wherein $R^3$ anionic units are selected from - $(CH_2)_pCO_2M$; $-(CH_2)_qSO_3M$; - $(CH_2)_qOSO_3M$; $-(CH_2)_qCH(SO_3M)-CH_2SO_3M$; - $(CH_2)_qCH(OSO_3M)CH_2OSO3M$; - $(CH_2)_qCH(SO_3M)CH_2SO_3M$; $-(CH_2)_nPO_3M$; $-PO_3M$ ;$-SO_3M$ and mixtures thereof; wherein M is hydrogen or a water soluble cation, preferably selected from sodium, potassium, ammonium, and mixtures thereof and in sufficient amount to satisfy charge balance;

x is from 5 to 50, preferably from 10 to 40, even more preferably from 15 to 30, most preferably from 20 to 25;

Q is a quaternizing unit selected from the group consisting of $C_1$-$C_{30}$ linear or branched alkyl, $C_6$-$C_{30}$ cycloalkyl, $C_7$-$C_{30}$ substituted or unsubstituted alkylenearyl, and mixtures thereof, preferably $C_1$-$C_{30}$ linear or branched alkyl, even more preferably $C_1$-$C_{10}$ or even $C_1$-$C_5$ linear or branched alkyl, most preferably methyl; the degree of quaternization preferably is more than 50%, more preferably more than 70%, even more preferably more than 90%, most preferably about 100;.

$X^-$ is an anion present in sufficient amount to provide electronic neutrality, preferably a water-soluble anion selected from the group consisting of chlorine, bromine, iodine, methyl sulfate, and mixtures thereof, more preferably chloride;

n is from 0 to 8, preferably 0 to 4, preferably 0 to 2, most preferably 0.

A suitable zwitterionic polyamine having the following general structure: $bis((C_2H_5O)(C_2H_4O)n)(CH_3)-N^+-C_xH_{2x}-N^+-(CH_3)-bis((C_2H_5O)(C_2H_4O)_n)$, wherein n = from 20 to 30, and x = from 3 to 8, or sulphated or sulphonated variants thereof.

A particular preferred zwitterionic polyamine is available from BASF as Lutensit Z96 polymer (zwitterionic hexamethylene diamine according to below formula: 100% quaternized and about 40% of the polyethoxy ($EO_{24}$) groups are sulfonated).

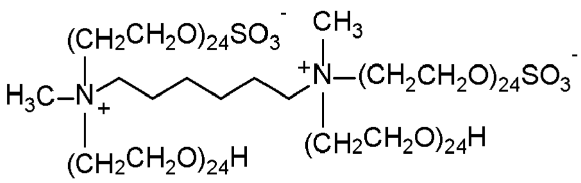

Another suitable zwitterionic polyamine is amphoterically-modified oligopropyleneimine ethoxylates as described in WO2021239547.

Other Polyester Soil Release Polymers.

The composition may comprise one or more other polyester soil release polymer (SRP). Preferably, the polyester SRP which comprise structure unit (I):

$$-[(OCHR^1-CHR^2)_a-O-OC-Ar-CO-]_b \qquad (I)$$

wherein:

a is from 1 to 200;

b is from 1 to 50;
Ar is independently selected from 1,4-substituted phenylene, and 1,3-substituted phenylene
$R^1$, $R^2$ are independently selected from H or $C_1$-$C_{18}$ n-alkyl or iso-alkyl; preferably selected from H or $C_1$ alkyl.

Optionally, the polymer further comprises one or more terminal group derived from polyalkylene glycolmonoalkylethers, preferably selected from structure below:

$$-O-[C_2H_4-O]_c-[C_3H_6-O]_d-[C_4H_8-O]_e-R^7$$

wherein:

$R^7$ is a linear or branched $C_{1-30}$ alkyl, $C_2$-$C_{30}$ alkenyl, or a cycloalkyl group with 5 to 9 carbon atoms, or a $C_8$-$C_{30}$ aryl group, or a $C_6$-$C_{30}$ arylalkyl group; preferably $C_{1-4}$ alkyl, more preferably methyl; and
c, d and e are, based on molar average, a number independently selected from 0 to 200, where the sum of c+d+e is from 2 to 500,

wherein the $[C_2H_4-O]$, $[C_3H_6-O]$ and $[C_4H_8-O]$ groups may be arranged blockwise, alternating, periodically and/or statistically, preferably blockwise and/or statistically, either of the $[C_2H_4-O]$, $[C_3H_6-O]$ and $[C_4H_8-O]$ groups can be linked to $-R^7$ and/or -O. Preferably, $[C_3H_6-O]$ group is linked to -O, and the -O is further connected to -OC-Ar-CO- via an ester bond.

Optionally, the polymer may comprises one or more anionic terminal unit (IV) and/or (V) as described in EP3222647. Where M is a counterion selected from $Na^+$, $Li^+$, $K^+$, ½ $Mg^{2+}$, ½ $Ca^{2+}$, 1/3 $Al^{3+}$, ammonium, mono-, di-, tri-, or tetraalkylammonium wherein the alkyl groups are $C_1$-$C_{18}$ alkyl or $C_2$-$C_{10}$ hydroxyalkyl, or mixtures thereof.

$$-O-CH_2-CH_2-SO_3M \qquad (IV)$$

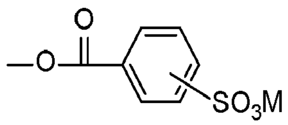
(V)

Optionally, the polymer may comprise crosslinking multifunctional structural unit which having at least three functional groups capable of the esterification reaction. The functional which may be for example acid -, alcohol -, ester -, anhydride - or epoxy groups, etc.

Optionally, other di- or polycarboxylic acids or their salts or their (di)alkylesters can be used in the polyesters of the invention, such as, 5-sulfoisophthalic acid, naphthalene-1,4-dicarboxylic acid, naphthalene-2,6,-dicarboxylic acid, tetrahydrophthalic acid, trimellitic acid, diphenoxyethane-4,4'-dicarboxylic acid, diphenyl-4,4'-dicarboxylic acid, 2,5-furandicarboxylic acid, adipic acid, sebacic acid, decan-1,10-dicarboxylic acid, fumaric acid, succinic acid, 1,4-cyclohexanedicarboxylic acid, cyclohexanediacetic acid, glutaric acid, azelaic acid, or their salts or their (di)alkyl esters, preferably their ($C_1$-$C_4$)-(di)alkyl esters and more preferably their (di)methyl esters, or mixtures thereof. Most preferred other di- or polycarboxylic acids are 5-sulfoisophthalic acid, 2,5-furandicarboxylic acid, salts or (di)alkyl esters thereof.

One type of preferred polyester SRPs are nonionic polyester SRP, which does not comprise any anionic monomer. A particular preferred nonionic terephthalate-derived SRP has a structure according to formula below:

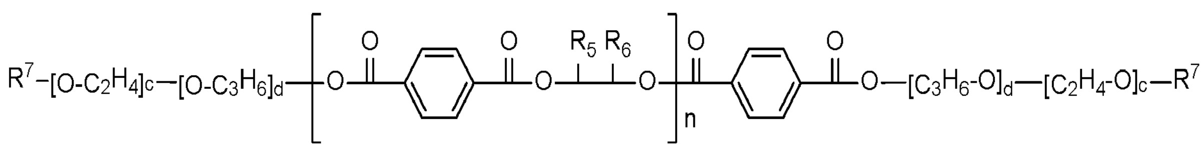

wherein:

$R_5$ and $R_6$ is independently selected from H or $CH_3$. More preferably, one of the $R_5$ and $R_6$ is H, and another is $CH_3$.
c, d are, based on molar average, a number independently selected from 0 to 200, where the sum of c+d is from 2 to 400, More preferably, d is from 0 to 50, c is from 1 to 200, More preferably, d is 1 to 10, c is 5 to

150,

$R^7$ is $C_1$-$C_4$ alkyl and more preferably methyl,

n is, based on molar average, from 1 to 50.

One example of most preferred above suitable terephthalate-derived nonionic SRP has one of the $R_5$ and $R_6$ is H, and another is $CH_3$; d is 0; c is from 5-100 and $R_7$ is methyl, and n is from 3-10.

Other suitable terephthalate-derived polyester SRP are described in patent WO2014019903, WO2014019658 and WO2014019659. The end capping group of these SRPs are selected from:

$$X\text{-}(OC_2H_4)_n\text{-}(OC_3H_6)_m\text{-}$$

wherein X is $C_1$-$C_4$ alkyl and preferably methyl, the -$(OC_2H_4)$ groups and the -$(OC_3H_6)$ groups are arranged blockwise and the block consisting of the -$(OC_3H_6)$ groups is bound to a COO group, n is based on a molar average a number of from 40 to 50, m is based on a molar average a number of from 1 to 10 and preferably of from 1 to 7.

Polyester soil release polymers may be available or convert into different forms, include powder, particle, liquid, waxy or premix. In some embodiment, other materials (for example, water, alcohol, other solvents, salt, surfactant, etc.) are needed to convert the polyester soil release polymer into different forms mentioned above, the wt% of active soil release polymer in the powder, particle, liquid, waxy or premix is in the range from 10% to 100%, for example 15%, 20%, 40%, 60%, 70%, 80%, 90%, 95%, 100%. Useful soil release polymer premix examples are described in EP351759 and WO2022100876. When the soil release polymers exist in liquid or premix from, the premix maybe transparent or opaque, white or slightly yellowish. Premix in opaque maybe use to provide an opaque appearance for the finish product or part of the finish product.

The polyester may or may not be biodegradable, preferred soil release polymers are readily biodegradable.

Example of suitable soil release polymers include TexCare® series supplied by Clariant, including noniconic soil release polymers Texcare® SRN 100, SRN 170, SRN 170 C, SRN 170 Terra, SRN 172, SRN 240, SRN 260, SRN 260 life, SRN 260 SG Terra, SRN UL50, SRN 300, SRN 325; and anionic soil release polymers TexCare® SRA 100, SRA 300, SRA300 F. Example of suitable soil release polymers also include REPEL-O-TEX® line of polymers supplied by Rhodia/Solvay, including nonionic soil release polymer REPEL-O-TEX® Crystal, Crystal PLUS, Crystal NAT, SRP6; and anionic soil release polymer REPEL-O-TEX® SF-2. Other example of commercial soil release polymers also includes WeylClean® series of soil release polymers supplied by WeylChem, including noniconic soil release polymers WeylClean® PLN1, PLN2; and anionic soil release polymers WeylClean® PSA1. Other examples of commercial soil release polymers are Marloquest® polymers, such as Marloquest® SL, HSCB, L235M, U, B, and G82, supplied by Sasol. Further suitable commercial soil release polymers include Sorez 100 (from ISP or Ashland).

Other Soil Release Polymers.

The composition may comprise one or more other types of soil release polymer (SRP).

Other suitable polymers also include a copolymer comprising N-isopropylacrylamide units, as described in WO2019197188, WO2019197187, WO2019197185, WO2019197186.

Polymers based on polysaccharide.

Various polysaccharides have proven to be useful starting material to make polymers for fabric and home care products, including cellulose, starch, guar, dextran, polyglucan, chitin, curdlan, xylose, Inulin, pullulan, locust bean gum, cassia gum, tamarind gum (xyloglucan), xanthan gum, amylose, amylopectin, scleroglucan and mixtures thereof.

The most common type of modified polysaccharide is modified cellulose.

Modified cellulose polymers include anionic modified cellulose polymers which been modified with functional groups that contain negative charge. Suitable anionic modified cellulose polymers include carboxyalkyl cellulose, such as carboxymethyl cellulose. In one preferred embodiment, the carboxymethyl cellulose has a degree of carboxymethyl substitution of from about 0.5 to about 0.9 and a molecular weight from about 80,000 Da to about 300,000 Da. Suitable carboxymethylcellulose is described in WO2011/031599 and WO2009/154933. Suitable carboxymethylcellulose include Finnfix® series sold by CP Kelco or Nouryon, which include Finnfix® GDA, a hydrophobically modified carboxymethylcellulose, e.g., the alkyl ketene dimer derivative of carboxymethylcellulose sold under the tradename Finnfix® SH1, or the blocky carboxymethylcellulose sold under the tradename Finnfix®V. Other suitable anionic modified cellulose polymers include sulphoalkyl group which described in WO2006117056, sulfoethyl cellulose which described in WO2014124872.

Modified cellulose polymers also include nonionic modified cellulose polymers which been modified by functional group that does not contain any charge. Suitable nonionic modified cellulose polymers include alkyl cellulose, hydroxyalkyl cellulose, hydroxyalkyl alkylcellulose, alkylalkoxyalkyl cellulose. Suitable nonionic modified cellulose polymers also

include nonionic cellulose carbamates which described in WO2015/044061; nonionic 6-desoxy-6-amino-celluloses derivative which described in US20180346846. Example of alkyl cellulose include methyl cellulose (MC), ethyl cellulose (EC), etc. Suitable ethyl cellulose are sold under tradename Ethocel™ by Dow Chemicals, DuPont, or IFF. Example of hydroxyalkyl cellulose include hydroxyethyl cellulose (HEC) and hydroxypropyl cellulose (HPC). Suitable HEC are sold under tradename Natrosol™ hydroxyethylcellulose by Ashland, such as Natrosol™ 250 with different grade available which has a total molar substitution (MS) of 2.5. Suitable HEC are also sold under tradename CELLOSIZE™ Hydroxyethyl Cellulose by Dow Chemicals. Suitable HPC are sold under tradename Klucel™ by Ashland. Example of hydroxyalkyl alkylcellulose include hydroxypropyl methylcellulose (HPMC), suitable HPMC are sold under tradename Methocel™ with different grade available by Dow Chemicals, DuPont or IFF, and under tradename Benecel™ by Ashland.

Another common type of modified polysaccharide is modified guar. Similar to modified cellulose, modified guar can be nonionic modified, and anionic modified. Suitable nonionic modified guar includes hydroxypropyl guar, such as N-Hance™ HP40 and HP40S guar available from Ashland. Suitable example of modified guar also include carboxymethyl hydroxypropyl guar (CMHPG) which is anionic and nonionic modified, such as Galactasol™ available from Ashland. Other nonionic and/or anionic modified guar include for example Jaguar® HP 105 (Hydroxypropyl Guar gum), Jaguar® SOFT and HP-120 COS (Carboxymethyl Hydroxypropyl Guar Gum).

Suitable modified polysaccharide polymers also include modified starch. Examples of modified starch include carboxylate ester of starch as described in WO2015144438, esterification product of starch with e.g. $C_6$-$C_{24}$ alk(en)yl succinic anhydride as described in EP0703243; starch maleates (starch react with maleic acid anhydride) as described US 6063914. Examples of modified starch also include, but not limit to, acetylated starch, acetylated distarch adipate, distarch phosphate, hydroxypropyl starch, hydroxy propyl distarch phosphate, phosphated distarch ohosphate, acetylated distarch phosphate, starch sodium octenyl succinate.

Suitable modified polysaccharide polymers also include polymers based on polyglucans. Suitable modified polyglucans are based on alpha 1,3-polyglucans and/or 1,6-polyglucans. In another embodiment, the modified polyglucans can be hydrophobic and/or hydrophilic modified, such as those described in WO2018112187, WO2019246228, WO2019246171, WO2021252558, WO2021252560, WO2021252561, EP3922704, WO2021252569, WO2021252562, WO2021252559, WO2021252575, WO2021252563. Along the hydrophobic and/or hydrophilic modified polyglucans, the polyglucan esters which described in WO2021252562, WO2021252559, WO2021252575, WO2021252563 are especially preferred due to their performance and biodegradability profiles.

Other suitable polysaccharide polymers also include those based on inulin. Example of modified inulin include carboxymethyl group modified inulin (CMI), suitable CMI are Carboxyline series sold by Cosun Beet Company, including Carboxyline 25-40D, Carboxyline 25 D Powder, Carboxyline 20 LS D Powder, Carboxyline 25, Carboxyline 25-30 UP.

Suitable modified polysaccharide polymers also include polymers based on other polysaccharide, such as xylose carbamates as described in US20210115358; carboxy or sulfo-alkylated pullulan as described in WO2019243072; carboxy- or sulfo-alkylated chitosan as described in WO2019/243108 and WO2021156093.

Polycarboxylate Polymers. The composition may also include one or more polycarboxylate polymers which comprise at least one carboxy group-containing monomer. The carboxy group-containing monomers are selected from acrylic acid, methacrylic acid, fumaric acid, maleic acid, itaconic acid, aconitic acid, mesaconic acid, citraconic acid, methylenemalonic acid, and salts thereof, and anhydride thereof.

Suitable polycarboxylate polymers include polyacrylate homopolymer having a molecular weight of from 4,000 Da to 9,000 Da, or from 6,000 Da to 9,000 Da. Other suitable carboxylate polymers include copolymer of acrylic acid (and/or methacrylic acid) and maleic acid having a molecular weight of from 50,000 Da to 120,000 Da, or from 60,000 Da to 80,000 Da. The polyacrylate homopolymer and copolymer of acrylic acid (and/or methacrylic acid) and maleic acid are commercially available as Acusol 445 and 445N, Acusol 531, Acusol 463, Acusol 448, Acusol 460, Acusol 465, Acusol 497, Acusol 490 from Dow Chemicals, and as Sokalan CP 5, Sokalan CP 7, Sokalan CP 45, and Sokalan CP 12S from BASF. Suitable polycarboxylate polymers also include polyitaconate homopolymers, such as Itaconix® DSP 2K™ sold by Itaconix, and Amaze SP available from Nouryon.

Suitable polycarboxylate polymers also include co-polymers comprising carboxy group-containing monomers and one or more sulfonate or sulfonic group-containing monomers. The sulfonate or sulfonic group containing monomers are selected rom 2-acrylamido-2-methyl-l-propanesulfonic acid (AMPS), 2-methacrylamido-2-methyl-l-propanesulfonic acid, 3-methacrylamido-2-hydroxy-propanesulfonic acid, ally sulfonic acid, methallysulfonic acid, 3-allyloxy-2-hydroxy-1-propanesulfonic acid, 2-methyl-2-propenen-l-sulfonic acid, styrenesulfonic acid, vinylsulfonic acid, 3-sulfopropyl acrylate, 3-sulfopropylmethacrylate, sulfomethylacrylamide, sulfomethylmethacrylamide and water soluble salts thereof. In one embodiment, suitable polymers comprise maleic acid, acrylic acid, and 3-allyloxy-2-hydroxy-1-propanesulfonic acid, such polymers are as described in US8450261 and US8389458. In another embodiment, suitable polymers comprise acrylic acid and 2-acrylamido-2-methylpropane sulfonate, such as those sold under tradename Acusol 588 by Dow Chemicals, Sokalan CP50 by BASF, Aquatreat AR-545, Versaflex 310 and Versaflex 310-37 by Nouryon. In another embodiment, suitable polymers also include Poly(itaconic acid-co-AMPS) sodium salt, such as Itaconix® TSI™ 322 and Itaconix® CHT™ 122 available from Itaconix.

Suitable polymer also includes those contain other structure units in addition to the sulfonate or sulfonic group group-containing monomers and carboxy group-containing monomers. Suitable polymer examples are described in WO2010024468 and WO2014/032267, the additional monomers herein are ether bond-containing monomers represented by formula (1) and (2) below:

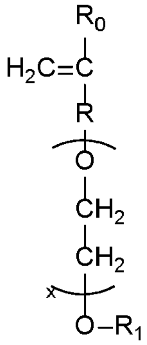

(1)

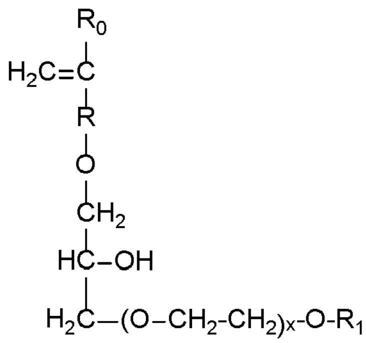

(2)

Wherein in Formula (1)

$R_0$ represents a hydrogen atom or $CH_3$ group,
R represents a $CH_2$ group, $CH_2CH_2$ group or single bond,
x represents a number 0-50, preferable 0-20, more preferable 0-5 (provided x represents a number 1-5 when R is a single bond), and
$R_1$ is a hydrogen atom or $C_1$ to $C_{20}$ organic group
Wherein in Formula (2),
$R_0$ represents a hydrogen atom or $CH_3$ group,
R represents a $CH_2$ group, $CH_2CH_2$ group or single bond,
x represents a number 0-5, and
$R_1$ is a hydrogen atom or $C_1$ to $C_{20}$ organic group.

A specific preferred polymer of this type comprises structure units derived from 1 to 49 wt% of 1-(allyloxy)-3-butoxypropan-2-ol, from 50 to 98 wt% acrylic acid or methacrylic acid, and from 1 to 49 wt% of 3-allyloxy-2-hydroxy-1-propanesulfonic acid, and the has a weight average molecular weight of from about 20,000 to about 60,000. a specific preferred polymer of this type comprises structure units derived from 1 to 10 wt% of 1-(allyloxy)-3-butoxypropan-2-ol, from 70 to 89 wt% acrylic acid or methacrylic acid, and from 10 to 20 wt% of 3-allyloxy-2-hydroxy-1-propanesulfonic acid, and the has a weight average molecular weight of from about 30,000 to about 60,000. Herein, 1-(allyloxy)-3-butoxypropan-2-ol is a preferred monomer as represented by formula (2) when $R_0$ is H, R is $CH_2$, x is 0, and $R_1$ is n-butyl ($C_4$-alkyl).

Suitable polycarboxylate polymers also include co-polymers comprising carboxy group-containing monomers and other suitable monomers. Other suitable monomers here are selected from esters and/or amide of the carboxy group-containing monomers, such as $C_1$-$C_{20}$ alkyl ester of acrylic acid; alkylene; vinyl ethers, such as methyl vinyl ether, styrene and any mixtures thereof. One specific preferred polymer family of this type is sold under tradename Gantrez by Ashland, which includes Gantrez An (alternating co-polymer of methyl vinyl ether and maleic anhydride), Gantrez S (alternating co-polymer of methyl vinyl ether and maleic acid), Gantrez ES (alternating co-polymer of methyl vinyl ether and maleic acid ester), Gantrez MS (alternating co-polymer of methyl vinyl ether and maleic acid salt).

Suitable polycarboxylate polymers also include polyepoxy succinic acid polymers (PESA). A most preferred polyepoxy succinic acid polymer can be identified using CAS number: 51274-37-4, or 109578-44-1. Suitable polyepoxy succinic acid polymers are commercially available from various suppliers, such as Aquapharm Chemicals Pvt. Ltd (commercial name: Maxinol 600); Shandong Taihe Water Treatment Technologies Co., Ltd (commercial name: PESA), and Sirius International (commercial name: Briteframe PESA).

Suitable polycarboxylate polymers also include polymer comprising a monomer having at least one aspartic acid group or a salt thereof, this polymer comprises at least 25 mol%, 40 mol%, or 50 mol%, of said monomer. A preferabed example is sodium salt of poly(aspartic acid) having a molecular weight of from 2000 to 3000 g/mol which is avilable as Baypure® DS 100 from Lanxess.

Other polymers. The composition may comprise block polymers of ethylene oxide, propylene oxide and butylene oxide. Examples of such block polymers include ethylene oxide-propylene oxide-ethylene oxide (EO/PO/EO) triblock copolymer, wherein the copolymer comprises a first EO block, a second EO block and PO block wherein the first EO block

and the second EO block are linked to the PO block. Blocks of ethylene oxide, propylene oxide, butylene oxide can also be arranged in other ways, such as (EO/PO) diblock copolymer, (PO/EO/PO) triblock copolymer. The block polymers may also contain additional butylene oxide (BO) block. Suitable block polymers are for example Pluronic PE series from BASF, including Pluronic PE3100, PE4300, PE6100, PE6200, PE6400, PE6800, PE8100, PE9200, PE9400, PE10100, PE10500, PE10400. Suitable block polymers also available as Tergitol L series from Dow Chemicals, such as Tergitol L-61, L-62, L-64, L-81, L-101. Due to the hydrophobic and hydrophilic nature, such block polymer sometime is also considered as nonionic surfactant in literature.

The composition may comprise dye transfer inhibiting agents (also called dye transfer inhibitor, or dye fixatives), which include, but are not limited to, polyvinylpyrrolidone polymers (PVP), poly(vinylpyridine-N-oxide) polymer (PVNO), poly(vinylimidazole), polyamine N-oxide polymers, copolymers of N-vinylpyrrolidone and N-vinylimidazole, polyvinyloxazolidones and polyvinylimidazoles or mixtures thereof. dye transfer inhibiting agents may be selected from the group consisting of reaction products of: i) polyamines with cyanamides and organic and/or inorganic acids, ii) cyanamides with aldehydes and ammonium salts, iii) cyanamides with aldehydes and amines, or iv) amines with epichlorohydrin.

The composition may comprise one or more other polymeric dispersing agents. Examples are poly (ethylene glycol), poly(vinyl alcohol).

Suitable polymers can also comprise monomers obtainable from renewable raw materials. Such monomers are described in US20200277548, US20200277549, WO2019096590.

Additional Amines: Additional amines may be used in the compositions described herein for added removal of grease and particulates from soiled materials. The compositions described herein may comprise from about 0.1% to about 10%, in some examples, from about 0.1% to about 4%, and in other examples, from about 0.1% to about 2%, by weight of the composition, of additional amines. Non-limiting examples of additional amines may include, but are not limited to, polyamines, oligoamines, triamines, diamines, pentamines, tetraamines, or combinations thereof. Specific examples of suitable additional amines include tetraethylenepentamine, triethylenetetraamine, diethylenetriamine, or a mixture thereof.

Bleaching Agents. It may be preferred for the composition to comprise one or more bleaching agents. Suitable bleaching agents other than bleaching catalysts include photobleaches, bleach activators, hydrogen peroxide, sources of hydrogen peroxide, pre-formed peracids and mixtures thereof. In general, when a bleaching agent is used, the compositions of the present invention may comprise from about 0.1% to about 50% or even from about 0.1% to about 25% bleaching agent or mixtures of bleaching agents by weight of the subject composition. Examples of suitable bleaching agents include:

(1) photobleaches for example sulfonated zinc phthalocyanine sulfonated aluminium phthalocyanines, xanthene dyes, thioxanthones, and mixtures thereof;

(2) pre-formed peracids: Suitable preformed peracids include, but are not limited to compounds selected from the group consisting of pre-formed peroxyacids or salts thereof typically a percarboxylic acids and salts, percarbonic acids and salts, perimidic acids and salts, peroxymonosulfuric acids and salts, for example, Oxone ®, and mixtures thereof.

Particularly preferred peroxyacids are phthalimido-peroxy-alkanoic acids, in particular ε-phthalimido peroxy hexanoic acid (PAP). Preferably, the peroxyacid or salt thereof has a melting point in the range of from 30°C to 60°C.

(3) sources of hydrogen peroxide, for example, inorganic perhydrate salts, including alkali metal salts such as sodium salts of perborate (usually mono- or tetra-hydrate), percarbonate, persulphate, perphosphate, persilicate salts and mixtures thereof. When employed, inorganic perhydrate salts are typically present in amounts of from 0.05 to 40 wt%, or 1 to 30 wt% of the overall fabric and home care product and are typically incorporated into such fabric and home care products as a crystalline solid that may be coated. Suitable coatings include, inorganic salts such as alkali metal silicate, carbonate or borate salts or mixtures thereof, or organic materials such as water-soluble or dispersible polymers, waxes, oils or fatty soaps; and

(4) bleach activators having R-(C=O)-L wherein R is an alkyl group, optionally branched, having, when the bleach activator is hydrophobic, from 6 to 14 carbon atoms, or from 8 to 12 carbon atoms and, when the bleach activator is hydrophilic, less than 6 carbon atoms or even less than 4 carbon atoms; and L is leaving group. Examples of suitable leaving groups are benzoic acid and derivatives thereof - especially benzene sulphonate. Suitable bleach activators include dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED) and nonanoyloxybenzene sulphonate (NOBS).

(5) Bleach Catalysts. The compositions of the present invention may also include one or more bleach catalysts capable of accepting an oxygen atom from a peroxyacid and/or salt thereof, and transferring the oxygen atom to an oxidizeable substrate. Suitable bleach catalysts include, but are not limited to: iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and alpha amino-ketones and mixtures thereof. One

particularly preferred catalyst is acyl hydrazone type such as 4-(2-(2-((2-hydroxyphenylmethyl)methylene)-hydrazinyl)-2-oxoethyl)-4-methylchloride.

(6) The composition may preferably comprise catalytic metal complexes. One preferred type of metal-containing bleach catalyst is a catalyst system comprising a transition metal cation of defined bleach catalytic activity, such as copper, iron, titanium, ruthenium, tungsten, molybdenum, or manganese cations.

If desired, the compositions herein can be catalyzed by means of a manganese compound. Such compounds and levels of use are well known in the art and include, for example, the manganese-based catalysts disclosed in U.S. 5,576,282. In some embodiments, an additional source of oxidant in the composition is not present, molecular oxygen from air providing the oxidative source.

Cobalt bleach catalysts useful herein are known, and are described, for example, in U.S. 5,597,936; U.S. 5,595,967.

Fluorescent Brightener: Commercial fluorescent brighteners suitable for the present disclosure can be classified into subgroups, including but not limited to: derivatives of stilbene, pyrazoline, coumarin, benzoxazoles, carboxylic acid, methinecyanines, dibenzothiophene-5,5-dioxide, azoles, 5- and 6-membered-ring heterocycles, and other miscellaneous agents.

The fluorescent brightener may be selected from the group consisting of disodium 4,4'-bis{ [4-anilino-6-morpholino-s-triazin-2-yl]-amino}-2,2'-stilbenedisulfonate (brightener 15, commercially available under the tradename Tinopal AMS-GX by BASF), disodium4,4'-bis{[4-anilino-6-(N-2-bis-hydroxyethyl)-s-triazine-2-yl]-amino}-2,2'-stilbenedisulonate (commercially available under the tradename Tinopal UNPA-GX by BASF), disodium 4,4'-bis{[4-anilino-6-(N-2-hydroxyethyl-N-methylamino)-s-triazine-2-yl]-amino}-2,2'-stilbenedisulfonate (commercially available under the tradename Tinopal 5BM-GX by BASF). More preferably, the fluorescent brightener is disodium 4,4'-bis{[4-anilino-6-morpholino-s-triazin-2-yl]-amino}-2,2'-stilbenedisulfonate or 2,2'-([1,1'-Biphenyl]-4,4'-diyldi-2,1-ethenediyl)bis-benzenesulfonic acid disodium salt. The brighteners may be added in particulate form or as a premix with a suitable solvent, for example nonionic surfactant, propanediol.

Fabric Hueing Agents: The compositions may comprise a fabric hueing agent (sometimes referred to as shading, bluing or whitening agents). Typically, the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof.

Chelating Agent. Preferably the composition comprises chelating agents and/or crystal growth inhibitor. Suitable molecules include copper, iron and/or manganese chelating agents and mixtures thereof. Suitable molecules include hydroxamic acids, aminocarboxylates, aminophosphonates, succinates, salts thereof, and mixtures thereof. Non-limiting examples of suitable chelants for use herein include ethylenediaminetetracetates, N-(hydroxyethyl)ethylenediaminetriacetates, nitrilotriacetates, ethylenediamine tetraproprionates, triethylenetetraaminehexacetates, diethylenetriaminepentaacetates, ethanoldiglycines, ethylenediaminetetrakis (methylenephosphonates), diethylenetriamine penta(methylene phosphonic acid) (DTPMP), ethylenediamine disuccinate (EDDS), hydroxyethanedimethylenephosphonic acid (HEDP), methylglycinediacetic acid (MGDA), diethylenetriaminepentaacetic acid (DTPA), N,N-Dicarboxymethyl glutamic acid (GLDA) and salts thereof, and mixtures thereof. Other nonlimiting examples of chelants of use in the present invention are found in U.S. Patent Nos. 7445644, 7585376 and 2009/0176684A1. Other suitable chelating agents for use herein are the commercial DEQUEST series, and chelants from Monsanto, DuPont, and Nalco, Inc. Yet other suitable chelants include the pyridinyl N Oxide type.

Encapsulates: The compositions may comprise an encapsulate. In some aspects, the encapsulate comprises a core, a shell having an inner and outer surface, where the shell encapsulates the core.

In certain aspects, the encapsulate comprises a core and a shell, where the core comprises a material selected from perfumes; brighteners; dyes; insect repellants; silicones; waxes; flavors; vitamins; fabric softening agents; skin care agents, e.g., paraffins; enzymes; antibacterial agents; bleaches; sensates; or mixtures thereof; and where the shell comprises a material selected from polyethylenes; polyamides; polyvinylalcohols, optionally containing other co-monomers; polystyrenes; polyisoprenes; polycarbonates; polyesters; polyacrylates; polyolefins; polysaccharides, e.g., alginate and/or chitosan; gelatin; shellac; epoxy resins; vinyl polymers; water insoluble inorganics; silicone; aminoplasts, or mixtures thereof. In some aspects, where the shell comprises an aminoplast, the aminoplast comprises polyurea, polyurethane, and/or polyureaurethane. The polyurea may comprise polyoxymethyleneurea and/or melamine formaldehyde.

Perfume. Preferred compositions of the invention comprise perfume. Typically the composition comprises a

perfume that comprises one or more perfume raw materials, selected from the group as described in WO08/87497. However, any perfume useful in a laundry care composition may be used. A preferred method of incorporating perfume into the compositions of the invention is via an encapsulated perfume particle comprising either a water-soluble hydroxylic compound or melamine-formaldehyde or modified polyvinyl alcohol.

Malodor Reduction Materials. The cleaning compositions of the present disclosure may comprise malodour reduction materials. Such materials are capable of decreasing or even eliminating the perception of one or more malodors. These materials can be characterized by a calculated malodor reduction value ("MORV"), which is calculated according to the test method shown in WO2016/049389.

As used herein "MORV" is the calculated malodor reduction value for a subject material. A material's MORV indicates such material's ability to decrease or even eliminate the perception of one or more malodors.

The cleaning compositions of the present disclosure may comprise a sum total of from about 0.00025% to about 0.5%, preferably from about 0.0025% to about 0.1%, more preferably from about 0.005% to about 0.075%, most preferably from about 0.01% to about 0.05%, by weight of the composition, of 1 or more malodor reduction materials. The cleaning composition may comprise from about 1 to about 20 malodor reduction materials, more preferably 1 to about 15 malodor reduction materials, most preferably 1 to about 10 malodor reduction materials.

One, some, or each of the malodor reduction materials may have a MORV of at least 0.5, preferably from 0.5 to 10, more preferably from 1 to 10, most preferably from 1 to 5. One, some, or each of the malodor reduction materials may have a Universal MORV, defined as all of the MORV values of >0.5 for the malodors tested as described herein. The sum total of malodor reduction materials may have a Blocker Index of less than 3, more preferable less than about 2.5, even more preferably less than about 2, and still more preferably less than about 1, and most preferably about 0. The sum total of malodor reduction materials may have a Blocker Index average of from about 3 to about 0.001.

In the cleaning compositions of the present disclosure, the malodor reduction materials may have a Fragrance Fidelity Index of less than 3, preferably less than 2, more preferably less than 1 and most preferably about 0 and/or a Fragrance Fidelity Index average of 3 to about 0.001 Fragrance Fidelity Index. As the Fragrance Fidelity Index decreases, the malodor reduction material(s) provide less and less of a scent impact, while continuing to counteract malodors.

The cleaning compositions of the present disclosure may comprise a perfume. The weight ratio of parts of malodor reduction composition to parts of perfume may be from about 1:20,000 to about 3000:1, preferably from about 1:10,000 to about 1,000:1, more preferably from about 5,000:1 to about 500:1, and most preferably from about 1:15 to about 1:1. As the ratio of malodor reduction composition to parts of perfume is tightened, the malodor reduction material(s) provide less and less of a scent impact, while continuing to counteract malodors.

Conditioning Agents: Suitable conditioning agents include high melting point fatty compounds. The high melting point fatty compound useful herein has a melting point of 25°C or higher and is selected from the group consisting of fatty alcohols, fatty acids, fatty alcohol derivatives, fatty acid derivatives, and mixtures thereof. Suitable conditioning agents also include nonionic polymers and conditioning oils, such as hydrocarbon oils, polyolefins, and fatty esters.

Suitable conditioning agents include those conditioning agents characterized generally as silicones (e.g., silicone oils, polyoils, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein. The compositions of the present invention may also comprise from about 0.05% to about 3% of at least one organic conditioning oil as the conditioning agent, either alone or in combination with other conditioning agents, such as the silicones (described herein). Suitable conditioning oils include hydrocarbon oils, polyolefins, and fatty esters.

Probiotics. The composition may comprise probiotics, such as those described in WO2009/043709.

Organic Acids. The detergent may comprise one or more organic acids selected from the group consisting of acetic acid, adipic acid, aspartic acid, carboxymethyloxymalonic acid, carboxymethyloxysuccinic acid, citric acid, formic acid, glutaric acid, hydroxyethyliminodiacetic acid, iminodiacetic acid, lactic acid, maleic acid, malic acid, malonic acid, oxydiacetic acid, oxydisuccinic acid, succinic acid, sulfamic acid, tartaric acid, tartaric-disuccinic acid, tartaric-monosuccinic acid, or mixtures thereof. Preferably, the detergent composition may comprise an organic acid selected from the group consisting of acetic acid, lactic acid, and citric acid.

Anti-oxidant: The composition may optionally contain an anti-oxidant present in the composition from about 0.001 to about 2% by weight. Preferably the antioxidant is present at a concentration in the range 0.01 to 0.08% by weight. Mixtures of anti-oxidants may be used.

Hygiene Agent: The compositions of the present invention may also comprise components to deliver hygiene and/or malodour benefits such as one or more of zinc ricinoleate, thymol, quaternary ammonium salts such as Bardac®, polyethylenimines (such as Lupasol® from BASF) and zinc complexes thereof, silver and silver compounds, especially those designed to slowly release Ag+ or nano-silver dispersions.

The cleaning compositions of the present invention may also contain antimicrobial agents. Preferably, the anti-microbial agent is selected from the group consisting of 4-4'-dichloro-2-hydroxy diphenyl ether ("Diclosan"), 2,4,4'-trichloro-2'-hydroxy diphenyl ether ("Triclosan"), and a combination thereof. Most preferably, the anti-microbial agent is

4-4'-dichloro-2-hydroxy diphenyl ether, commercially available from BASF, under the trademark name Tinosan®HP100.

Pearlescent Agent: Non-limiting examples of pearlescent agents include: mica; titanium dioxide coated mica; bismuth oxychloride; fish scales; mono and diesters of alkylene glycol. The pearlescent agent may be ethyleneglycoldistearate (EGDS).

Opacifier: In one embodiment, the composition might also comprise an opacifier. As the term is used herein, an "opacifier" is a substance added to a material in order to make the ensuing system opaque. In one preferred embodiment, the opacifier is Acusol, which is available from Dow Chemicals. Acusol opacifiers are provided in liquid form at a certain % solids level. As supplied, the pH of Acusol opacifiers ranges from 2.0 to 5.0 and particle sizes range from 0.17 to 0.45 um. In one preferred embodiment, Acusol OP303B and 301 can be used.

In yet another embodiment, the opacifier may be an inorganic opacifier. Preferably, the inorganic opacifier can be $TiO_2$, ZnO, talc, $CaCO_3$, and combination thereof. The composite opacifier-microsphere material is readily formed with a preselected specific gravity, so that there is little tendency for the material to separate.

Solvents: The solvent system in the present compositions can be a solvent system containing water alone or mixtures of organic solvents either without or preferably with water. The compositions may optionally comprise an organic solvent. Suitable organic solvents include $C_4$-$C_{14}$ ethers and diethers, glycols, alkoxylated glycols, $C_6$-$C_{16}$ glycol ethers, alkoxylated aromatic alcohols, aromatic alcohols, aliphatic branched alcohols, alkoxylated aliphatic branched alcohols, alkoxylated linear $C_1$-$C_5$ alcohols, linear $C_1$-$C_5$ alcohols, amines, $C_8$-$C_{14}$ alkyl and cycloalkyl hydrocarbons and halohydrocarbons, and mixtures thereof. Preferred organic solvents include 1,2-propanediol, 2,3 butane diol, ethanol, glycerol, ethoxylated glycerol, dipropylene glycol, methyl propane diol and mixtures thereof 2 ethyl hexanol, 3,5,5,trimethyl-1 hexanol, and 2 propyl heptanol. Solvents may be a polyethylene or polypropylene glycol ether of glycerin. Other lower alcohols, $C_1$-$C_4$ alkanolamines such as monoethanolamine and triethanolamine, can also be used. Solvent systems can be absent, for example from anhydrous solid embodiments of the invention, but more typically are present at levels in the range of from about 0.1% to about 98%, preferably at least about 1% to about 50%, more usually from about 5% to about 25%, alternatively from about 1% to about 10% by weight of the liquid detergent composition of said organic solvent. These organic solvents may be used in conjunction with water, or they may be used without water

Hydrotrope: The composition may optionally comprise a hydrotrope in an effective amount, i.e. from about 0% to 15%, or about 1% to 10% , or about 3% to about 6%, so that compositions are compatible in water. Suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903.

Suds Suppressor. Compounds for reducing or suppressing the formation of suds can be incorporated into the water-soluble unit dose articles. Suds suppression can be of particular importance in the so-called "high concentration cleaning process" and in front-loading style washing machines. Examples of suds supressors include monocarboxylic fatty acid and soluble salts therein, high molecular weight hydrocarbons such as paraffin, fatty acid esters (e.g., fatty acid triglycerides), fatty acid esters of monovalent alcohols, aliphatic $C_{18}$-$C_{40}$ ketones (e.g., stearone), N-alkylated amino triazines, waxy hydrocarbons preferably having a melting point below about 100 °C, silicone suds suppressors, and secondary alcohols. Preferred fatty acid blends may be mixtures enriched or Fatty acid mixtures enriched with 2-alkyl fatty acid, preferably 2-methyl octanoic acid

Additional suitable antifoams are those derived from phenylpropylmethyl substituted polysiloxanes.

The detergent composition may comprise a suds suppressor selected from organomodified silicone polymers with aryl or alkylaryl substituents combined with silicone resin and a primary filler, which is modified silica. The detergent compositions may comprise from about 0.001% to about 4.0%, by weight of the composition, of such a suds suppressor.

The detergent composition comprises a suds suppressor selected from: a) mixtures of from about 80 to about 92% ethylmethyl, methyl(2-phenylpropyl) siloxane; from about 5 to about 14% MQ resin in octyl stearate; and from about 3 to about 7% modified silica; b) mixtures of from about 78 to about 92% ethylmethyl, methyl(2-phenylpropyl) siloxane; from about 3 to about 10% MQ resin in octyl stearate; from about 4 to about 12% modified silica; or c) mixtures thereof, where the percentages are by weight of the anti-foam.

Liquid Laundry Detergent Composition. The fabric and home care product can be a laundry detergent composition, such as a liquid laundry detergent composition. Suitable liquid laundry detergent compositions can comprise a non-soap surfactant, wherein the non-soap surfactant comprises an anionic non-soap surfactant and a non-ionic surfactant. The laundry detergent composition can comprise from 10% to 60%, or from 20% to 55% by weight of the laundry detergent composition of the non-soap surfactant. The non-soap anionic surfactant to nonionic surfactant are from 1:1 to 20: 1, from 1.5: 1 to 17.5: 1, from 2: 1 to 15: 1, or from 2.5:1 to 13: 1. Suitable non-soap anionic surfactants include linear alkylbenzene sulphonate, alkyl sulphate or a mixture thereof. The weight ratio of linear alkylbenzene sulphonate to alkyl sulphate can be from 1:2 to 9: 1, from 1:1 to 7:1, from 1:1 to 5:1, or from 1: 1 to 4:1. Suitable linear alkylbenzene sulphonates are $C_{10}$-$C_{16}$ alkyl benzene sulfonic acids, or $C_{11}$-$C_{14}$ alkyl benzene sulfonic acids. Suitable alkyl sulphate anionic surfactants include alkoxylated alkyl sulphates, non-alkoxylated alkyl sulphates, and mixture thereof. Preferably, the HLAS surfactant comprises greater than 50% $C_{12}$, preferably greater than 60%, preferably greater than 70% $C_{12}$, more

preferably greater than 75% $C_{12}$. Suitable alkoxylated alkyl sulphate anionic surfactants include ethoxylated alkyl sulphate anionic surfactants. Suitable alkyl sulphate anionic surfactants include ethoxylated alkyl sulphate anionic surfactant with a mol average degree of ethoxylation of from 1 to 5, from 1 to 3, or from 2 to 3. The alkyl alkoxylated sulfate may have a broad alkoxy distribution or a peaked alkoxy distribution. The alkyl portion of the AES may include, on average, from 13.7 to about 16 or from 13.9 to 14.6 carbons atoms. At least about 50% or at least about 60% of the AES molecule may include having an alkyl portion having 14 or more carbon atoms, preferable from 14 to 18, or from 14 to 17, or from 14 to 16, or from 14 to 15 carbon atoms. The alkyl sulphate anionic surfactant may comprise a non-ethoxylated alkyl sulphate and an ethoxylated alkyl sulphate wherein the mol average degree of ethoxylation of the alkyl sulphate anionic surfactant is from 1 to 5, from 1 to 3, or from 2 to 3. The alkyl fraction of the alkyl sulphate anionic surfactant can be derived from fatty alcohols, oxo-synthesized alcohols, Guerbet alcohols, or mixtures thereof. Preferred alkyl sulfates include optionally ethoxylated alcohol sulfates including 2-alkyl branched primary alcohol sulfates especially 2-branched $C_{12}$-$C_{15}$ primary alcohol sulfates, linear primary alcohol sulfates especially linear $C_{12}$-$C_{14}$ primary alcohol sulfates, and mixtures thereof. The laundry detergent composition can comprise from 10% to 50%, or from 15% to 45%, or from 20% to 40%, or from 30% to 40% by weight of the laundry detergent composition of the non-soap anionic surfactant.

Suitable non-ionic surfactants can be selected from alcohol broad or narrow range alkoxylates, an oxo-synthesised alcohol alkoxylate, Guerbet alcohol alkoxylates, alkyl phenol alcohol alkoxylates, or a mixture thereof. The laundry detergent composition can comprise from 0.01% to 10%, from 0.01% to 8%, from 0.1% to 6%, or from 0.15% to 5% by weight of the liquid laundry detergent composition of a non-ionic surfactant.

The laundry detergent composition comprises from 1.5% to 20%, or from 2% to 15%, or from 3% to 10%, or from 4% to 8% by weight of the laundry detergent composition of soap, such as a fatty acid salt. Such soaps can be amine neutralized, for instance using an alkanolamine such as monoethanolamine.

The laundry detergent composition can comprises an adjunct ingredient selected from the group comprising builders including citrate, enzymes, bleach, bleach catalyst, dye, hueing dye, Leuco dyes, brightener, cleaning polymers including alkoxylated polyamines and polyethyleneimines, amphiphilic copolymers, soil release polymer, surfactant, solvent, dye transfer inhibitors, chelant, diamines, perfume, encapsulated perfume, polycarboxylates, structurant, pH trimming agents, antioxidants, antibacterial, antimicrobial agents, preservatives and mixtures thereof.

The laundry detergent composition can have a pH of from 2 to 11, or from 6.5 to 8.9, or from 7 to 8, wherein the pH of the laundry detergent composition is measured at a 10% product concentration in demineralized water at 20°C.

The liquid laundry detergent composition can be Newtonian or non-Newtonian, preferably non-Newtonian.

For liquid laundry detergent compositions, the composition can comprise from 5% to 99%, or from 15% to 90%, or from 25% to 80% by weight of the liquid detergent composition of water.

Structured Liquids: In some embodiments of the invention, the composition is in the form of a structured liquid. Such structured liquids can either be internally structured, whereby the structure is formed by primary ingredients (e.g. surfactant material) and/or externally structured by providing a three dimensional matrix structure using secondary ingredients (e.g. polymers, clay and/or silicate material), for use e.g. as thickeners. The composition may comprise a structurant, preferably from 0.01wt% to 5wt%, from 0.1wt% to 2.0wt% structurant. Examples of suitable structurants are given in US2006/0205631A1, US2005/0203213A1, US7294611, US6855680. The structurant is typically selected from the group consisting of diglycerides and triglycerides, ethylene glycol distearate, microcrystalline cellulose, cellulose-based materials, microfiber cellulose, hydrophobically modified alkali-swellable emulsions such as Polygel W30 (3VSigma), biopolymers, xanthan gum, gellan gum, hydrogenated castor oil, derivatives of hydrogenated castor oil such as non-ethoxylated derivatives thereof and mixtures thereof, in particular, those selected from the group of hydrogenated castor oil, derivatives of hydrogenated castor oil, microfibullar cellulose, hydroxyfunctional crystalline materials, long chain fatty alcohols, 12-hydroxystearic acids, clays and mixtures thereof. One preferred structurant is described in US Patent No. 6,855,680 which defines suitable hydroxyfunctional crystalline materials in detail. Preferred is hydrogenated castor oil. Some structurants have a thread-like structuring system having a range of aspect ratios. Another preferred structurant is based on cellulose and may be derived from a number of sources including biomass, wood pulp, citrus fibers and the like.

Pouches. In a preferred embodiment of the invention, the composition is provided in the form of a unitized dose, either tablet form or preferably in the form of a liquid/solid (optionally granules)/gel/paste held within a water-soluble film in what is known as a pouch or pod. The composition can be encapsulated in a single or multi-compartment pouch. Multi-compartment pouches are described in more detail in EP-A-2133410. When the composition is present in a multi-compartment pouch, the composition of the invention may be in one or two or more compartments, thus the dye may be present in one or more compartments, optionally all compartments. Non-shading dyes or pigments or other aesthetics may also be used in one or more compartments. In one embodiment the composition is present in a single compartment of a multi-compartment pouch.

Preferred film materials are polymeric materials. The film material can be obtained, for example, by casting, blow-molding, extrusion or blown extrusion of the polymeric material, as known in the art. Preferred polymers, copolymers or derivatives thereof suitable for use as pouch material are selected from polyvinyl alcohols, polyvinyl pyrrolidone, polyalkylene oxides, acrylamide, acrylic acid, cellulose, cellulose ethers, cellulose esters, cellulose amides, polyvinyl

acetates, polycarboxylic acids and salts, polyaminoacids or peptides, polyamides, polyacrylamide, copolymers of maleic/acrylic acids, polysaccharides including starch and gelatine, natural gums such as xanthum and carragum. More preferred polymers are selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, and most preferably selected from polyvinyl alcohols, polyvinyl alcohol copolymers and hydroxypropyl methyl cellulose (HPMC), and combinations thereof. Preferably, the level of polymer in the pouch material, for example a PVA polymer, is at least 60%. The polymer can have any weight average molecular weight, preferably from about 1000 to 1,000,000, more preferably from about 10,000 to 300,000 yet more preferably from about 20,000 to 150,000. Mixtures of polymers can also be used as the pouch material. This can be beneficial to control the mechanical and/or dissolution properties of the compartments or pouch, depending on the application thereof and the required needs. Suitable mixtures include for example mixtures wherein one polymer has a higher water-solubility than another polymer, and/or one polymer has a higher mechanical strength than another polymer. Also suitable are mixtures of polymers having different weight average molecular weights, for example a mixture of PVA or a copolymer thereof of a weight average molecular weight of about 10,000-40,000, preferably around 20,000, and of PVA or copolymer thereof, with a weight average molecular weight of about 100,000 to 300,000, preferably around 150,000. Also, suitable herein are polymer blend compositions, for example comprising hydrolytically degradable and water-soluble polymer blends such as polylactide and polyvinyl alcohol, obtained by mixing polylactide and polyvinyl alcohol, typically comprising about 1-35% by weight polylactide and about 65% to 99% by weight polyvinyl alcohol. Preferred for use herein are polymers which are from about 60% to about 98% hydrolysed, preferably about 80% to about 90% hydrolysed, to improve the dissolution characteristics of the material.

Naturally, different film material and/or films of different thickness may be employed in making the compartments of the present invention. A benefit in selecting different films is that the resulting compartments may exhibit different solubility or release characteristics. Most preferred film materials are PVA films known under the MonoSol trade reference M8630, M8900, H8779 and those described in US 6 166 117 and US 6 787 512 and PVA films of corresponding solubility and deformability characteristics.

The film material herein can also comprise one or more additive ingredients. For example, it can be beneficial to add plasticizers, for example glycerol, ethylene glycol, diethyleneglycol, propylene glycol, sorbitol and mixtures thereof. Other additives include functional detergent additives to be delivered to the wash water, for example organic polymeric dispersants, etc.

The following is an exemplary water-soluble unit dose formulation. The composition can be part of a single chamber water soluble unit dose article or can be split over multiple compartments resulting in below "averaged across compartments" full article composition. The composition is enclosed within a polyvinyl alcohol based water soluble, the polyvinyl alcohol comprising a blend of a polyvinyl alcohol homopolymer and an anionic e.g. carboxylated polyvinyl alcohol copolymer.

| Ingredients | (wt%) |
|---|---|
| Fatty alcohol ethoxylate non-ionic surfactant, $C_{12-14}$ average degree of ethoxylation of 7 | 3.8 |
| Lutensol XL100 | 0.5 |
| Linear $C_{11-14}$ alkylbenzene sulphonate | 24.6 |
| AE3S Ethoxylated alkyl sulphate with an average degree of ethoxylation of 3 | 12.5 |
| Citric acid | 0.7 |
| Palm Kernel Fatty acid | 5.3 |
| Nuclease enzyme* (wt% active protein) | 0.01 |
| Protease enzyme (wt% active protein) | 0.07 |
| Amylase enzyme (wt% active protein) | 0.005 |
| Xyloglucanese enzyme (wt% active protein) | 0.005 |
| Mannanase enzyme (wt% active protein) | 0.003 |
| Ethoxylated polyethyleneimine (Lutensol FP620 - $PEI_{600}EO_{20}$) | 1.4 |
| Amphiphilic graft copolymer** | 1.6 |
| Zwitterionic polyamine (Lutensit Z96) | 1.5 |
| Polyester of the present invention | 0.6 |

(continued)

| Ingredients | (wt%) |
|---|---|
| Polysaccharide-based cationic polymer of the present invention | 0.6 |
| HEDP | 2.2 |
| Brightener 49 | 0.4 |
| Silicone anti-foam | 0.3 |
| Hueing dye | 0.05 |
| 1,2 PropaneDiol | 11.0 |
| Glycerine | 4.7 |
| DPG (DiPropyleneGlycol) | 1.7 |
| TPG (TriPropyleneGlycol) | 0.1 |
| Sorbitol | 0.1 |
| Monoethanolamine | 10.2 |
| $K_2SO_3$ | 0.4 |
| $MgCl_2$ | 0.3 |
| water | 10.5 |
| Hydrogenated castor oil | 0.1 |
| Perfume | 2.1 |
| Aesthetic dye & Minors | Balance to 100 |
| pH (10% product concentration in demineralized water at 20°C) | 7.4 |
| Description of super-scripts:<br>*Nuclease enzyme is as claimed in co-pending European application 19219568.3<br>**polyethylene glycol graft polymer comprising a polyethylene glycol backbone (Pluriol E6000) and hydrophobic vinyl acetate side chains, comprising 40% by weight of the polymer system of a polyethylene glycol backbone polymer and 60% by weight of the polymer system of the grafted vinyl acetate side chains | |

Solid Form. As noted previously, the laundry care compositions may be in a solid form. Suitable solid forms include tablets and particulate forms, for example, granular particles, flakes or sheets. Various techniques for forming detergent compositions in such solid forms are well known in the art and may be used herein.

The following is an exemplary solid free-flowing particulate laundry detergent composition.

| Ingredients | (wt%) |
|---|---|
| Anionic detersive surfactant (such as alkyl benzene sulphonate, alkyl ethoxylated sulphate and mixtures thereof) | from 8wt% to 15wt% |
| Non-ionic detersive surfactant (such as alkyl ethoxylated alcohol) | from 0.1wt% to 4wt% |
| Cationic detersive surfactant (such as quaternary ammonium compounds) | from 0wt% to 4wt% |
| Other detersive surfactant (such as zwiterionic detersive surfactants, amphoteric surfactants and mixtures thereof) | from 0wt% to 4wt% |
| Carboxylate polymer (such as co-polymers of maleic acid and acrylic acid and/or carboxylate polymers comprising ether moieties and sulfonate moieties) | from 0.1wt% to 4wt% |
| Polyethylene glycol polymer (such as a polyethylene glycol polymer comprising polyvinyl acetate side chains) | from 0wt% to 4wt% |
| Polyester of the present invention | from 0.1wt% to 4wt% |
| Polysaccharide-based cationic polymer of the present invention | from 0.1wt% to 4wt% |

(continued)

| Ingredients | (wt%) |
|---|---|
| Cellulosic polymer (such as carboxymethyl cellulose, methyl cellulose and combinations thereof) | from 0.5wt% to 2wt% |
| Zeolite builder and phosphate builder (such as zeolite 4A and/or sodium tripolyphosphate) | from 0wt% to 4wt% |
| Other co-builder (such as sodium citrate and/or citric acid) | from 0wt% to 3wt% |
| Carbonate salt (such as sodium carbonate and/or sodium bicarbonate) | from 0wt% to 20wt% |
| Silicate salt (such as sodium silicate) | from 0wt% to 10wt% |
| Filler (such as sodium sulphate and/or bio-fillers) | from 10wt% to 70wt% |
| Source of hydrogen peroxide (such as sodium percarbonate) | from 0wt% to 20wt% |
| Bleach activator (such as tetraacetylethylene diamine (TAED) and/or nonanoyloxybenzenesulphonate (NOBS)) | from 0wt% to 8wt% |
| Bleach catalyst (such as oxaziridinium-based bleach catalyst and/or transition metal bleach catalyst) | from 0wt% to 0.1wt% |
| Other bleach (such as reducing bleach and/or pre-formed peracid) | from 0wt% to 10wt% |
| Photobleach (such as zinc and/or aluminium sulphonated phthalocyanine) | from 0wt% to 0.1wt% |
| Chelant (such as ethylenediamine-N'N'-disuccinic acid (EDDS) and/or hydroxyethane diphosphonic acid (HEDP)) | from 0.2wt% to 1wt% |
| Hueing agent (such as direct violet 9, 66, 99, acid red 50, solvent violet 13 and any combination thereof) | from 0wt% to 1wt% |
| Brightener (C.I. fluorescent brightener 260 or C.I. fluorescent brightener 351) | from 0.1wt% to 0.4wt% |
| Protease (such as Savinase, Savinase Ultra, Purafect, FN3, FN4 and any combination thereof) | from 0.1wt% to 0.4wt% |
| Amylase (such as Termamyl, Termamyl ultra, Natalase, Optisize, Stainzyme, Stainzyme Plus and any combination thereof) | from 0wt% to 0.2wt% |
| Cellulase (such as Carezyme and/or Celluclean) | from 0wt% to 0.2wt% |
| Lipase (such as Lipex, Lipolex, Lipoclean and any combination thereof) | from 0wt% to 1wt% |
| Other enzyme (such as xyloglucanase, cutinase, pectate lyase, mannanase, bleaching enzyme) | from 0wt% to 2wt% |
| Fabric softener (such as montmorillonite clay and/or polydimethylsiloxane (PDMS)) | from 0wt% to 15wt% |
| Flocculant (such as polyethylene oxide) | from 0wt% to 1wt% |
| Suds suppressor (such as silicone and/or fatty acid) | from 0wt% to 4wt% |
| Perfume (such as perfume microcapsule, spray-on perfume, starch encapsulated perfume accords, perfume loaded zeolite, and any combination thereof) | from 0.1wt% to 1wt% |
| Aesthetics (such as coloured soap rings and/or coloured speckles/noodles) | from 0wt% to 1wt% |
| Miscellaneous | balance to 100wt% |

Fibrous Water-soluble Unit Dose Article As used herein, the phrases "water-soluble unit dose article," "water-soluble fibrous structure", and "water-soluble fibrous element" mean that the unit dose article, fibrous structure, and fibrous element are miscible in water. In other words, the unit dose article, fibrous structure, or fibrous element is capable of forming a homogeneous solution with water at ambient conditions. "Ambient conditions" as used herein means 23°C ± 1.0°C and a relative humidity of 50% ± 2%. The water-soluble unit dose article may contain insoluble materials, which are dispersible in aqueous wash conditions to a suspension mean particle size that is less than about 20 microns, or less than about 50 microns.

The fibrous water-soluble unit dose article may include any of the disclosures found in U.S. Patent Application No. 15/880,594 filed on January 26, 2018; U.S. Patent Application No. 15/880,599 filed January 26, 2018; and U.S. Patent

Application No. 15/880,604 filed January 26, 2018; incorporated by reference in their entirety. Preferred water-soluble fibrous structure comprises particles having a ratio of Linear Alkylbenzene Sulfonate to Alkylethoxylated Sulfate or Alkyl Sulfate of greater than 1.

These fibrous water-soluble unit dose articles can be dissolved under various wash conditions, e.g., low temperature, low water and/or short wash cycles or cycles where consumers have been overloading the machine, especially with items having high water absorption capacities, while providing sufficient delivery of active agents for the intended effect on the target consumer substrates (with similar performance as today's liquid products). Furthermore, the water-soluble unit dose articles described herein can be produced in an economical manner by spinning fibers comprising active agents. The water-soluble unit dose articles described herein also have improved cleaning performance.

Method of Use. The compositions of this invention, prepared as hereinbefore described, can be used to form aqueous washing/treatment solutions for use in the laundering/treatment of fabrics. Generally, an effective amount of such compositions is added to water, for example in a conventional fabric automatic washing machine, to form such aqueous laundering solutions. The aqueous washing solution so formed is then contacted, typically under agitation, with the fabrics to be laundered/treated therewith. An effective amount of the liquid detergent compositions herein added to water to form aqueous laundering solutions can comprise amounts sufficient to form from about 500 to 7,000 ppm of composition in aqueous washing solution, or from about 1,000 to 3,000 ppm of the laundry care compositions herein will be provided in aqueous washing solution.

Typically, the wash liquor is formed by contacting the laundry care composition with wash water in such an amount so that the concentration of the laundry care composition in the wash liquor is from above 0g/l to 5g/l, or from 1g/l, and to 4.5g/l, or to 4.0g/l, or to 3.5g/l, or to 3.0g/l, or to 2.5g/l, or even to 2.0g/l, or even to 1.5g/l. The method of laundering fabric or textile may be carried out in a top-loading or front-loading automatic washing machine or can be used in a hand-wash laundry application. In these applications, the wash liquor formed and concentration of laundry detergent composition in the wash liquor is that of the main wash cycle. Any input of water during any optional rinsing step(s) is not included when determining the volume of the wash liquor.

The wash liquor may comprise 40 liters or less of water, or 30 liters or less, or 20 liters or less, or 10 liters or less, or 8 liters or less, or even 6 liters or less of water. The wash liquor may comprise from above 0 to 15 liters, or from 2 liters, and to 12 liters, or even to 8 liters of water. Typically, from 0.01kg to 2kg of fabric per liter of wash liquor is dosed into said wash liquor. Typically, from 0.01kg, or from 0.05kg, or from 0.07kg, or from 0.10kg, or from 0.15kg, or from 0.20kg, or from 0.25kg fabric per liter of wash liquor is dosed into said wash liquor. Optionally, 50g or less, or 45g or less, or 40g or less, or 35g or less, or 30g or less, or 25g or less, or 20g or less, or even 15g or less, or even 10g or less of the composition is contacted to water to form the wash liquor. Such compositions are typically employed at concentrations of from about 500 ppm to about 15,000 ppm in solution. When the wash solvent is water, the water temperature typically ranges from about 5 °C to about 90 °C and, when the situs comprises a fabric, the water to fabric ratio is typically from about 1:1 to about 30:1. Typically the wash liquor comprising the laundry care composition of the invention has a pH of from 3 to 11.5.

In one aspect, such method comprises the steps of optionally washing and/or rinsing said surface or fabric, contacting said surface or fabric with any composition disclosed in this specification then optionally washing and/or rinsing said surface or fabric is disclosed, with an optional drying step.

Drying of such surfaces or fabrics may be accomplished by any one of the common means employed either in domestic or industrial settings. The fabric may comprise any fabric capable of being laundered in normal consumer or institutional use conditions, and the invention is suitable for cellulosic substrates and in some aspects also suitable for synthetic textiles such as polyester and nylon and for treatment of mixed fabrics and/or fibers comprising synthetic and cellulosic fabrics and/or fibers. As examples of synthetic fabrics are polyester, nylon, these may be present in mixtures with cellulosic fibers, for example, polycotton fabrics. The solution typically has a pH of from 7 to 11, more usually 8 to 10.5. The compositions are typically employed at concentrations from 500 ppm to 5,000 ppm in solution. The water temperatures typically range from about 5°C to about 90°C. The water to fabric ratio is typically from about 1:1 to about 30:1.

Another method includes contacting a nonwoven substrate, which is impregnated with the detergent composition, with a soiled material. As used herein, "nonwoven substrate" can comprise any conventionally fashioned nonwoven sheet or web having suitable basis weight, caliper (thickness), absorbency, and strength characteristics. Non-limiting examples of suitable commercially available nonwoven substrates include those marketed under the trade names SONTARA® by DuPont and POLY WEB® by James River Corp.

Carbon Source of Raw Materials:

The raw materials for preparation of the surfactant, polymers and other ingredients can be based on fossil carbon or renewable carbon. Renewable carbon is a carbon source that avoid the use of fossil carbon such as natural gas, coal, petroleum. Typically, renewable carbon is derived from the biomass, carbon capture, or chemical recycling.

Biomass is a renewable carbon source formed through photosynthesis in the presence of sunlight, or chemosynthesis process in the absence of sunlight. In some cases, polymers isolated from biomass can be used directly, or

further derivatized to make performance polymers. For example, the use of polysaccharide (such as starch) and derivatized polysaccharide (such as cellulose derivatives, guar derivatives, dextran derivatives) in fabric home care composition are known. In some cases, biomass can be converted into basic chemicals under certain thermal, chemical, or biological conditions. For example, bioethanol can be derived from biomass such as straw, and further convert to biobased polyethylene glycol. Other nonlimiting examples of renewable carbon from biomass include plants (e.g., sugar cane, beets, corn, potatoes, citrus fruit, woody plants, lignocellulosics, hemicellulosics, cellulosic waste), animals, animal fats, fish, bacteria, fungi, plant-based oils, and forestry products. These resources can be naturally occurring, hybrids, or genetically engineered organisms.

Carbon capture is another renewable carbon source which use various process to capture $CO_2$ or methane from industrial or natural processes, or directly from air (direct capture). Captured methane and $CO_2$ maybe converted into syngas, and/or further convert to basic chemicals, including but not limit to methanol, ethanol, fatty alcohols such as $C_{12}/C_{14}$ or even $C_{16}/C_{18}$ alcohols, other alcohols, olefins, alkanes, saturated and unsaturated organic acids, etc. These basic chemicals can used as or further convert to monomers for making transformed to usable chemicals by e.g. catalytic processes, such as the Fischer-Tropsch process or by fermentation by $C_1$ -fixing microorganisms.

Chemical recycling is another renewable carbon source which allow plastics from waste management industry to be recycled and converted into base chemicals and chemical feedstocks. In some cases, waste plastics which cannot be re-used or mechanical recycled are convert to hydrocarbons or basic petrochemicals through gasification, pyrolysis or hydrothermal treatment processes, the hydrocarbons and basic petrochemicals can be further convert into monomers for polymers. In some cases, waste plastics are depolymerized into monomers to make new polymers. It is also possible that waste plastics are depolymerized into oligomers, the oligomers can be used as building blocks to make new polymers. The waste plastic converted by various processes to a waste plastic feedstock for the above materials may either be used alone or in combination with traditional surfactant feedstocks, such as kerosene, polyolefins derived from natural gas, coal, crude oil or even biomass, or waste fat/oil-derived paraffin and olefin, to produce biodegradable surfactants for use in detergents and other industries (thereby providing a benefit to society).

Preferably, the surfactant, polymers and other ingredients contains renewable carbon, the Renewable Carbon Index (RCI, a measure of sustainability by dividing the number of carbons derived from renewable sources by the total number of carbons in an active ingredient) of the polymer is above 10%, more preferably above 30%, more preferably above 50%, more preferably above 60%, more preferably between 70% to 100% (including 100%), and most preferably 100%.

EXAMPLES

The examples below are intended to illustrate the invention in detail without, however, limiting it thereto. Unless explicitly stated otherwise, all percentages given are percentages by weight (% by wt. or wt.-%).

Polyester preparation

General procedure for the preparation of the polyesters of the examples.

The polyester synthesis is carried out by the reaction of dimethyl terephthalate (DMT), dimethyl-5-sulfoisophthalate sodium salt (5-SIM), 1,2-propylene glycol, ethylene glycol, alkyl capped polyalkylene glycol (mono hydroxyl-functional polyalkylene glycol monoalkyl ether), and optionally polyalkylene glycol, using sodium acetate (NaOAc) and tetraisopropyl orthotitanate (IPT) as the catalyst system. The synthesis is a two-step procedure. The first step is a trans-esterification and the second step is a polycondensation.

Key to reactants or ingredients used in the examples:

| | |
|---|---|
| 5-SIM | is dimethyl-5-sulfoisophthalate sodium salt |
| AE NI | is alkyl ethoxylate $(EO)_7$ |
| AES | is alcohol ethoxysulfate |
| DMT | is dimethyl terephthalate |
| EG | is ethylene glycol |
| HEDP | is 1-hydroxyethane-1,1-diphosphonic acid |
| IPT | is tetraisopropyl orthotitanate |
| LAS | is linear alkyl benzene sulphonate |
| MGDA | is methylglycine-diacetic acid |
| mPEG2000 | is mono hydroxyl-functional polyethylene glycol monomethyl ether, average molecular weight 2000 g/mol |
| mPEG3000 | is mono hydroxyl-functional polyethylene glycol monomethyl ether, average molecular weight 3000 |

| | |
|---|---|
| | g/mol |
| mPEG4000 | is mono hydroxyl-functional polyethylene glycol monomethyl ether, average molecular weight 4000 g/mol |
| MetEG | methoxytetraethylene glycol |
| NaOAc | is sodium acetate |
| PEG300 | is di-hydroxyl-functional polyethylene glycol), average molecular weight 300 g/mol |
| PG | is 1,2-propylene glycol |

Inventive polyester example 1:

83.22 g (0.42 mol) of dimethyl terephthalate (DMT), 42.3 g (0.14 mol) of dimethyl-5-sulfoisophthalate sodium salt (5-SIM), 40.05 g (0.53 mol) of 1,2-propylene glycol (PG), 34.60 g (0.56 mol) of ethylene glycol (EG), 200 g (0.10 mol) of mPEG2000 and 0.5 g of sodium acetate (NaOAc) (anhydrous) are weighed into a reaction vessel at room temperature. For the melting process and homogenization, the mixture is heated up to 110 - 120 °C. 200 µL of tetraisopropyl orthotitanate (IPT) is added and the mixture is further heated up to 210 °C over 3 hours sparged by a nitrogen stream. During the transesterification methanol is released from the reaction and is distilled out of the system. Once the head-temperature is below 55 °C, nitrogen is switched off and the pressure is reduced to 10 mbar. PG and EG are distilled out of the system. The mixture is stirred for further 4 hours at a pressure of 10 mbar. The reaction mixture is cooled down to 140 - 150 °C. Vacuum is released with nitrogen and the polyester is transferred out of the reactor.

Inventive polyester examples 2 to 4 are synthesized according to similar procedure as inventive polyester example 1 with monomer type and dosage described in Table 1.

Inventive polyester example 5:

58.26 g (0.30 mol) of DMT, 29.63 g (0.10 mol) of 5-SIM, 28.04 g (0.37 mol) of PG, 24.19 g (0.39 mol) of EG, 10.50 g (0.04 mol) of PEG300, 140 g (0.07 mol) of mPEG2000 and 0.38 g of NaOAc (anhydrous) are weighed into a reaction vessel at room temperature. For the melting process and homogenization, the mixture is heated up to 110 - 120 °C. 134 µL of IPT is added and the mixture is further heated up to 210 °C over 3 hours sparged by a nitrogen stream. During the transesterification methanol is released from the reaction and is distilled out of the system. Once the head-temperature is below 55 °C, nitrogen is switched off and the pressure is reduced to 10 mbar. PG and EG are distilled out of the system. The mixture is stirred for further 4 hours at a pressure of 10 mbar. The reaction mixture is cooled down to 140 - 150 °C. Vacuum is released with nitrogen and the polyester is transferred out of the reactor. The average number of moles of polyalkylene glycol PEG300 per mole of polyester is 1.0.

Comparative polyester example 1:

116.50 g (0.59 mol) of DMT, 59.25 g (0.20 mol) of 5-SIM, 56.07 g (0.74 mol) of PG, 48.40 g (0.78 mol) of EG, 29.10 g (0.14 mol) of methoxytetraethylene glycol (MetEG) and 0.5 g of NaOAc (anhydrous) are weighed into a reaction vessel at room temperature. For the melting process and homogenization, the mixture is heated up to 110 - 120 °C. 200 µL of IPT is added and the mixture is further heated up to 210 °C over 3 hours sparged by a nitrogen stream. During the transesterification methanol is released from the reaction and is distilled out of the system. Once the head-temperature is below 55 °C, nitrogen is switched off and the pressure is reduced to 10 mbar. PG and EG are distilled out of the system. The mixture is stirred for further 4 hours at a pressure of 10 mbar. The reaction mixture is cooled down to 140 - 150 °C. Vacuum is released with nitrogen and the polyester is transferred out of the reactor.

Table 1 Monomer type and dosage for the preparation of inventive and comparative polyesters.

| Monomer | Inventive polyester | | | | | Comparative polyester |
|---|---|---|---|---|---|---|
| | 1 | 2 | 3 | 4 | 5 | 1 |
| DMT (g) | 83.22 | 58.26 | 29.13 | 27.55 | 58.26 | 116.50 |
| 5-SIM (g) | 42.3 | 29.63 | 14.81 | 14.01 | 29.63 | 59.25 |
| PG (g) | 40.05 | 28.04 | 14.02 | 0.00 | 28.04 | 56.07 |
| EG (g) | 34.60 | 24.19 | 12.10 | 22.25 | 24.19 | 48.40 |
| PEG300 (g) | - | - | - | - | 10.50 | - |

(continued)

| Monomer | Inventive polyester | | | | | Comparative polyester |
|---|---|---|---|---|---|---|
| | 1 | 2 | 3 | 4 | 5 | 1 |
| Type of terminal unit (endcap) | mPEG2000 | mPEG3000 | mPEG4000 | mPEG2000 | mPEG2000 | MetEG |
| terminal unit (g) | 200 | 210 | 140 | 66.19 | 140 | 29.10 |
| NaOAc (g) | 0.50 | 0.38 | 0.19 | 0.10 | 0.38 | 0.5 |
| IPT ($\mu$L) | 200 | 134 | 80 | 40 | 134 | 200 |

The average amount of the monomers in a polyester is calculated as 1) a polyester has end-caps on both sides, 2) used DMT and 5-SIM are equally integrated into a polyester and 3) the excess amount of PG and EG are equally distilled out of the system.

Method of Testing biodegradability of polyesters:

The biodegradability of polyesters is determined following the OECD 301B Ready Biodegradability $CO_2$ Evolution Test Guideline. In this study, the test substance is the sole carbon and energy source and under aerobic conditions microorganisms metabolize the test substance producing $CO_2$ or incorporating the carbon into biomass. The amount of $CO_2$ produced by the test substance (corrected for the $CO_2$ evolved by the blank inoculum) is expressed as a percentage of the theoretical amount of $CO_2$ ($ThCO_2$) that could have been produced if the organic carbon in the test substance was completely converted to $CO_2$.

Method of Evaluating Whiteness Benefit of polyesters

During the laundry process, soil which has been removed from dirty clothes is suspended in the detergent solution. Some suspended soil can redeposit back onto clothes. Whiteness maintenance performance is the ability of a detergent to keep white items from whiteness loss when they are washed in the presence of soils.

The whiteness benefit of polyesters is evaluated using automatic Tergotometer with 10 pots for laundry formulation testing. SBL2004 test soil strips supplied by WFK Testgewebe GmbH are used to simulate consumer soil levels. On average, every 1 SBL2004 strip is loaded with 8g soil. The SBL2004 test soil strips were cut into 5×5 cm squares for use in the test. White Fabric swatches below from WFK Testgewebe GmbH are typically used as whiteness tracers. Codes for the used fabric are summarized in Table 2.

Table 2 Codes for fabrics

| Code | Fiber Content | % Fiber Content | Fabric Construction | Size |
|---|---|---|---|---|
| CK | Cotton | 100 | Weft Knit | (5×5cm) |
| PC | Polyester/cotton | 65/35 | Weave | (5×5cm) |
| PE | Polyester | 100 | Weft Knit | (5×5cm) |
| PS | Polyester/SpandexTM | 95/5 | Weft Knit | (5×5cm) |

Additional ballast (background fabric swatches) is also used to simulate a fabric load and provide mechanical energy during the real laundry process. Ballast loads are comprised of cotton and poly cotton knit swatches at 5×5 cm size.

4 cycles of wash are needed to complete the test:

Cycle 1: Desired amount of detergent is fully dissolved by mixing with 1L water (at defined hardness) in each tergotometer pot. 60 grams of fabrics, including whiteness tracers (4 types, each with 4 replicates), 30 pieces 5×5 cm SBL2004, and ballast are washed and rinsed in the tergotometer pot under defined conditions.

Cycle 2: The whiteness tracers and ballast from each pot are then washed and rinsed again together with a new set of SBL2004 (5×5cm, 30 pieces) following the process of cycle 1. All other conditions remain the same as in cycle 1.

Cycle 3: The whiteness tracers and ballast from each pot are then washed and rinsed again together with a new set of SBL2004 (5×5cm, 30 pieces) following the process of cycle 1. All other conditions remain the same as in cycle 1.

Cycle 4: The whiteness tracers and ballast from each pot are then washed and rinsed again together with a new set of SBL2004 (5×5cm, 30 pieces) following the process of cycle 1. All other conditions remain the same as in cycle 1.

After Cycle 4, all whiteness tracers & ballast are tumble dried between 60-65°C until dry, then the WI(CIE) of the dry tracers is measured using Konica Minolta CM-3610D spectrophotometer.

Whiteness Performance (in water-soluble unit dose detergent)

Inventive and comparative water-soluble unit dose liquid composition prepared by traditional means known to those of ordinary skill in the art by mixing the listed ingredients (Table 3).

The whiteness performance of compositions are evaluated according to the method described above, the wash concentration of the composition is 1984ppm, the concentration of water-soluble film is 47ppm, wash temperature is 35°C, water hardness is 20gpg. Composition CC1 is used as reference to test the impact of polyesters and cationic polymers.

Table 3 Ingredients of compositions

| Ingredients (wt.-%) | Inventive and comparative composition |
|---|---|
| LAS | 24.52 |
| AES | 12.62 |
| AENI | 2.02 |
| Suds Suppressor | 0.30 |
| HEDP | 2.23 |
| Monoethanolamine | 8.10 |
| 1,2-Propylene Glycol | 12.72 |
| Dipropyleneglycol | 0.84 |
| $K_2SO_3$ | 0.35 |
| $MgCl_2$ | 0.32 |
| Citric Acid | 0.70 |
| Fatty Acid | 1.61 |
| Glycerol | 4.76 |
| Brightener | 0.04 |
| Blue dye | 0.0014 |
| Enzyme (including Protease, Amylase, and Mannanase) | 0.087 |
| Preservative | 0.009 |
| Hydrogenated castor oil | 0.09 |
| Perfume | 2.05 |
| Inventive polyester 1 | As defined in specific composition |
| Inventive polyester 2 | As defined in specific composition |
| Inventive polyester 4 | As defined in specific composition |
| Comparative polyester 1 | As defined in specific composition |
| Cationic polymer | As defined in specific composition |
| Water / minors | Balance |
| [a] Fabric: 100% Polyester Knit (PE). | |

In a study focus on Inventive polyester 2, ΔWI(CIE) of compositions CC2-CC5 (Comparative) and IC1 (Inventive) vs CC1 (Comparative) are reported in Table 4. Composition IC1 comprising combination of inventive polyester 2 and cationic polymer (Cationic modified HEC polymer) shows stronger whiteness performance than composition CC5

comprising combination of comparative polyester 1 and cationic polymer (Cationic modified HEC polymer). In comparation, composition CC2 and CC3 which comprising only inventive or comparative polyester and no cationic polymer, show similar performance.

Table 4 Ingredients of compositions CC1-CC5 and IC1 and ΔWI(CIE)-value for composition CC2-CC5 and IC1 versus composition CC1

| Ingredients (wt.-%) | CC1 (reference) | CC2 | CC3 | CC4 | CC5 | IC1 |
|---|---|---|---|---|---|---|
| Inventive polyester 2 | - | - | 1.00 | - | - | 1.00 |
| Comparative polyester 1 | - | 1.00 | - | - | 1.00 | - |
| Cationic modified HEC polymer | - | - | - | 1.00 | 1.00 | 1.00 |
| ΔWI(CIE) vs Reference [a] | 0 | +13.3 | +13.9 | -2.1 | -4.7 | +13.8 |
| [a] Fabric: 100% Polyester Knit (PE). | | | | | | |

In a separate study focus on Inventive polyester 1 and 4, ΔWI(CIE) of compositions CC6-CC9 (comparative) and IC2-IC3 (inventive) versus composition CC1 (comparative) are reported in Table 5. Composition IC2 and IC3 comprising combination of inventive polyester 1 or 4 and cationic polymer (Cationic modified HEC polymer) show stronger whiteness performance than composition CC9 comprising combination of comparative polyester 1 and cationic polymer (Cationic modified HEC polymer). In comparation, composition CC6, CC7 and CC8 which comprising only inventive or comparative polyester and no cationic polymer, show similar performance.

Table 5 Ingredients of compositions CC1, CC6-CC9 and IC2-IC3 and ΔWI(CIE)-value for composition CC6-CC9 and IC2-IC3 versus composition CC1

| Ingredients (wt.-%) | CC1 (Reference) | CC6 | CC7 | CC8 | CC9 | IC2 | IC3 |
|---|---|---|---|---|---|---|---|
| Inventive polyester 1 | - | - | 1.00 | - | - | 1.00 | - |
| Inventive polyester 4 | - | - | - | 1.00 | - | - | 1.00 |
| Comparative polyester 1 | - | 1.00 | - | - | 1.00 | - | - |
| Cationic modified HEC polymer | - | - | - | - | 1.00 | 1.00 | 1.00 |
| ΔWI(CIE) vs Reference [a] | 0 | +27.2 | +27.6 | +26.6 | +15.5 | +26.8 | +27.4 |
| ΔWI(CIE) vs Reference [b] | 0 | +6.1 | +6.2 | +4.6 | -0.9 | +6.1 | +4.6 |
| [a] Fabric: 100% Polyester Knit (PE).<br>[b] Fabric: 95%/5% Polyester/Spandex | | | | | | | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

**1.** A fabric and home care composition comprising:

(a) a fabric and home care ingredient;
(b) an anionic polyester soil release polymer; and
(c) a polysaccharide-based cationic polymer,

wherein the anionic polyester soil release polymer comprises:

(A) one or more structural units of the formula (I)

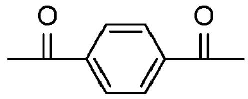

(I)

and

(B) one or more structural units of the formula (II)

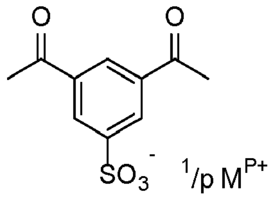

(II)

wherein

$^{1}/_{p}\ M^{p+}$ is a cation, preferably selected from the group consisting of monovalent cations $M^+$ (p = 1), divalent cations ½ $M^{2+}$ (p = 2) and trivalent cations ⅓ $M^{3+}$ (p = 3) and more preferably selected from the group consisting of $H^+$, $Li^+$, $Na^+$, $K^+$, ½ $Mg^{2+}$, ½ $Ca^{2+}$, ⅓ $Al^{3+}$, $NH_4^+$ and $R^aR^bR^cR^dN^+$, wherein $R^a$, $R^b$, $R^c$ and $R^d$, independently of one another, are H, linear or branched, preferably linear, $(C_1-C_{22})$-alkyl groups or linear or branched, preferably linear, $(C_2-C_{10})$-hydroxyalkyl groups, and wherein in the cations $R^aR^bR^cR^dN^+$ at least one of $R^a$, $R^b$, $R^c$ and $R^d$ is not H,

and

(C) one or more structural units of the formula (III)

$$-O-R^1-O- \qquad (III)$$

wherein

$R^1$ is a linear or branched alkylene group represented by the formula $(C_mH_{2m})$ wherein m is an integer from 2 to 12, preferably from 2 to 6, more preferably from 2 to 4 and even more preferably from 2 to 3, most preferably 3,

and

(D) one or more terminal groups of the formula (IV)

$$-O-[C_nH_{2n}-O]_x-R^2 \qquad (IV)$$

wherein

$R^2$ is a linear or branched $C_1$-$C_{30}$ alkyl group, a cycloalkyl group with 5 to 9 carbon atoms or a $C_6$-$C_{30}$ arylalkyl group, preferably a linear or branched $C_1$-$C_{30}$ alkyl group, more preferably a linear $C_1$-$C_6$ alkyl group and even more preferably $CH_3$,
n is 2 or an integer > 2, preferably is an integer from 2 to 12, more preferably is an integer from 2 to 6 and even more preferably is an integer from 2 to 4, whereby the definition of n may vary within a single terminal group of the formula (IV), and
x is, based on molar average, a number of at least 30, preferably from 30 to 200, more preferably from 40 to 180, even more preferably from 50 to 150, particularly preferably from 60 to 120 and extraordinarily preferably from 65 to 115.

**2.** The composition according to claim 1, wherein the terminal group of the formula (IV) has the structure:

$$-O-[C_nH_{2n}-O]_x-R^2 \qquad (IV)$$

wherein

$R^2$ is a linear $C_1$-$C_6$ alkyl group and even more preferably $CH_3$,
n is an integer from 2 to 4, and
x is, based on molar average, a number from 50 to 150, particularly preferably from 60 to 120 and extraordinarily preferably from 65 to 115.

**3.** The composition according to any preceding claim, wherein the structural unit of formula (III) has a structure according to (III-A):

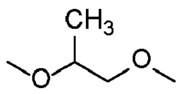

or

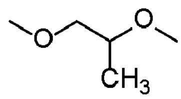

(III-A)

**4.** The composition according to any preceding claim, wherein the polysaccharide-based cationic polymer comprises a backbone selected from cellulose, starch, glycogen, guar, dextran, polyglucan, chitin, pectin, curdlan, xylose, inulin, pullulan, locust bean gum, cassia gum, tamarind gum (xyloglucan), xanthan gum, amylose, amylopectin, scleroglucan, and any combination thereof.

**5.** The composition according to any preceding claim, wherein the polysaccharide-based cationic polymer is selected from cationic modified cellulose, cationic modified dextran, cationic modified polyglucan, cationic modified guar, cationic modified inulin, cationic modified starch, and any combination thereof.

**6.** The composition according to any preceding claim 1, wherein the polysaccharide-based cationic polymer is selected from cationic modified cellulose, preferably cationic modified hydroxyethyl cellulose polymer.

**7.** The composition according to any preceding claim, wherein the composition comprises from 0.01wt% to 5.0wt% of the polysaccharide-based cationic polymer.

**8.** The composition according to any preceding claim, wherein the composition comprises from 0.01wt% to 5.0wt% of the anionic soil release polymer.

**9.** The composition according to any preceding claim, wherein the composition comprises from 1.0wt% to 70wt% detersive surfactant.

**10.** The composition according to any preceding claim, wherein the composition further comprises one or more depositable conditioning active selected from the group consisting of a softening active, a freshness active, or a combination thereof,

wherein the softening active, if present, is selected from the group consisting of quaternary ammonium ester compounds, silicones, non-ester quaternary ammonium, compounds, amines, fatty esters, sucrose esters, silicones, dispersible polyolefins, polysaccharides, fatty acids, softening or conditioning oils, polymer latexes, or combinations thereof, preferably a quaternary ammonium ester compounds, silicones, or combinations thereof; and
wherein the freshness active, if present, is selected from the group consisting of free perfume, pro-perfume, a perfume delivery system, malodor control agent, or mixtures thereof, preferably free perfume, a perfume delivery system, or mixtures thereof.

**11.** The composition according to any preceding claim, wherein the composition is a laundry detergent composition, and wherein the fabric and home care ingredient is an anionic detersive surfactant.

**12.** Use of the composition according to any of claims 1-11 to reduce the adhesion of soil to a fabric surface.

**13.** Use of the composition according to any of claims 1-11 to reduce the adhesion of biological stains or microorganisms to textiles.

**14.** Use of the composition according to any of claims 1-11 to control malodor.

**15.** Use of the composition according to any of claims 1-12 to deposit one or more depositable conditioning active on surfaces.

Europäisches Patentamt
European Patent Office
Office européen des brevets

# EUROPEAN SEARCH REPORT

Application Number
EP 23 20 7441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 327 106 A1 (HENKEL AG & CO KGAA [DE]) 30 May 2018 (2018-05-30)<br>* paragraphs [0024]-[0041], [0048]-[0060], [0074], [0076] *<br>----- | 1-15 | INV.<br>C11D3/00<br>C11D3/37<br>C11D3/22<br>C11D3/48 |
| Y | US 4 702 857 A (GOSSELINK EUGENE P [US]) 27 October 1987 (1987-10-27)<br>* Table III; column 31, lines 1-27; column 30, lines 1-25; column 8, lines 11-25 *<br>----- | 1-15 | |
| Y | WO 2008/110318 A2 (CLARIANT INT LTD [CH]; DUECKER BARBARA [DE] ET AL.)<br>18 September 2008 (2008-09-18)<br>* claims 1, 2, 18 *<br>----- | 1-15 | |
| Y | US 2008/234165 A1 (PANANDIKER RAJAN KESHAV [US] ET AL) 25 September 2008 (2008-09-25)<br>* paragraphs [0100]-[0108] and [0223] *<br>----- | 1-15 | |

TECHNICAL FIELDS SEARCHED (IPC)

C11D

The present search report has been drawn up for all claims

1

| Place of search | Date of completion of the search | Examiner |
|---|---|---|
| The Hague | 24 April 2024 | Engelskirchen, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 20 7441

This annex lists the patent family members relating to the patent documents cited in the above-mentioned European search report. The members are as contained in the European Patent Office EDP file on
The European Patent Office is in no way liable for these particulars which are merely given for the purpose of information.

24-04-2024

| Patent document cited in search report | | Publication date | Patent family member(s) | | | Publication date |
|---|---|---|---|---|---|---|
| EP 3327106 | A1 | 30-05-2018 | NONE | | | |
| US 4702857 | A | 27-10-1987 | NONE | | | |
| WO 2008110318 | A2 | 18-09-2008 | DE | 102007013217 | A1 | 18-09-2008 |
| | | | WO | 2008110318 | A2 | 18-09-2008 |
| US 2008234165 | A1 | 25-09-2008 | AR | 067228 | A1 | 07-10-2009 |
| | | | BR | PI0808649 | A2 | 12-08-2014 |
| | | | CA | 2680151 | A1 | 25-09-2008 |
| | | | EP | 2126017 | A1 | 02-12-2009 |
| | | | JP | 2010520350 | A | 10-06-2010 |
| | | | US | 2008234165 | A1 | 25-09-2008 |
| | | | WO | 2008114171 | A1 | 25-09-2008 |
| | | | ZA | 200906152 | B | 26-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019111948 A
- WO 2019111949 A
- WO 2019111946 A
- WO 2019111947 A
- WO 2022060754 A
- WO 2021242942 A
- WO 2020091988 A
- WO 2021194808 A
- WO 2015095358 A
- WO 2021225837 A
- WO 2021257786 A
- WO 2021257793 A
- WO 2021257932 A
- WO 2023287684 A
- US 20190274943 A
- US 20180119055 A
- WO 2004067737 A
- WO 2015091989 A
- WO 2015091990 A
- WO 2015024739 A
- WO 2015143360 A
- US 6312936 B1
- US 5679630 A
- US 4760025 A
- DE 102006022216 A1
- DE 102006022224 A1
- WO 2015089447 A
- WO 2015089441 A
- WO 2016066756 A
- WO 2016066757 A
- WO 2016069557 A
- WO 2016069563 A
- WO 2016069569 A
- WO 2017089093 A
- WO 2020156419 A
- WO 8906270 A
- WO 05052161 A
- WO 05052146 A
- WO 07044993 A2
- WO 2014194032 A
- WO 2014194054 A
- WO 2014194117 A
- WO 2015193488 A
- WO 2016075078 A
- WO 9217577 A
- US 5352604 A
- US 7153818 B
- WO 9700324 A
- EP 1022334 A
- WO 9402597 A
- WO 9418314 A
- WO 9623874 A
- WO 9743424 A
- US 5856164 A
- WO 9923211 A
- WO 9623873 A
- WO 0060060 A
- WO 06002643 A
- US 6093562 A
- WO 09149130 A
- WO 2016091688 A
- WO 2014099523 A
- WO 2014164777 A
- WO 2009149271 A
- US 6939702 B1
- US 20090217464 A
- WO 2013171241 A
- WO 2011084412 A
- WO 2013033318 A
- WO 2018228880 A
- WO 2018228881 A
- EP 3299457 A
- WO 2018209026 A
- WO 2017036901 A
- WO 2017005798 A
- US 4435307 A
- US 5648263 A
- US 5691178 A
- US 5776757 A
- US 7361736 B
- US 6268197 B
- US 6630340 B
- WO 2002077242 A
- US 7172891 B
- WO 2017162836 A
- WO 2018108865 A
- WO 2018011277 A
- WO 2018178061 A
- WO 2020074499 A
- WO 2018184873 A
- WO 2017186936 A
- WO 2017186937 A
- WO 2017186943 A
- WO 2017207770 A
- WO 2019086520 A
- WO 2019086528 A
- WO 2019086530 A
- WO 2019086532 A

- WO 2019086521 A
- WO 2019086526 A
- WO 2020002604 A
- WO 2020002608 A
- WO 2020007863 A
- WO 2020007875 A
- WO 2020008024 A
- WO 2020070063 A
- WO 2020070249 A
- WO 2020088957 A
- WO 2020088958 A
- WO 2020207944 A
- WO 2015040159 A
- WO 2015185689 A
- WO 2007138053 A
- WO 2021160795 A
- WO 2021160851 A
- WO 2020005476 A
- WO 2020030760 A
- WO 2020030469 A
- WO 2009061990 A
- WO 2006108857 A
- WO 2021061774 A
- WO 2021239547 A
- EP 3222647 A
- WO 2014019903 A
- WO 2014019658 A
- WO 2014019659 A
- EP 351759 A
- WO 2022100876 A
- WO 2019197188 A
- WO 2019197187 A
- WO 2019197185 A
- WO 2019197186 A
- WO 2011031599 A
- WO 2009154933 A
- WO 2006117056 A
- WO 2014124872 A
- WO 2015044061 A
- US 20180346846 A
- WO 2015144438 A
- EP 0703243 A
- US 6063914 A
- WO 2018112187 A
- WO 2019246228 A
- WO 2019246171 A
- WO 2021252558 A
- WO 2021252560 A
- WO 2021252561 A
- EP 3922704 A
- WO 2021252569 A
- WO 2021252562 A
- WO 2021252559 A
- WO 2021252575 A
- WO 2021252563 A
- US 20210115358 A
- WO 2019243072 A
- WO 2019243108 A
- WO 2021156093 A
- US 8450261 B
- US 8389458 B
- WO 2010024468 A
- WO 2014032267 A
- US 20200277548 A
- US 20200277549 A
- WO 2019096590 A
- US 5576282 A
- US 5597936 A
- US 5595967 A
- US 7445644 B
- US 7585376 B
- US 20090176684 A1
- WO 0887497 A
- WO 2016049389 A
- WO 2009043709 A
- US 3915903 A
- US 20060205631 A1
- US 20050203213 A1
- US 7294611 B
- US 6855680 B
- EP 2133410 A
- US 6166117 A
- US 6787512 B
- EP 19219568
- US 88059418
- US 88059918
- US 88060418

**Non-patent literature cited in the description**

- *CHEMICAL ABSTRACTS*, 83855-79-2
- *CHEMICAL ABSTRACTS*, 51274-37-4
- *CHEMICAL ABSTRACTS*, 109578-44-1